(19) Europäisches Patentamt / European Patent Office / Office européen des brevets

(11) **EP 4 255 599 B1**

(12) **EUROPEAN PATENT SPECIFICATION**

(45) Date of publication and mention
of the grant of the patent:
**20.08.2025 Bulletin 2025/34**

(21) Application number: **21816110.7**

(22) Date of filing: **03.12.2021**

(51) International Patent Classification (IPC):
**B01D 3/14** *(2006.01)*    **C07C 29/70** *(2006.01)*
**C07C 31/30** *(2006.01)*    **B01D 3/00** *(2006.01)*

(52) Cooperative Patent Classification (CPC):
(C-Sets available)
**B01D 3/009; B01D 3/143; C07C 29/70;** Y02P 20/10
(Cont.)

(86) International application number:
**PCT/EP2021/084140**

(87) International publication number:
**WO 2022/117803 (09.06.2022 Gazette 2022/23)**

(54) **INTEGRATED PROCESS FOR THE PARALLEL PRODUCTION OF ALKALI METAL METHOXIDES**

INTEGRIERTES VERFAHREN ZUR PARALLELEN HERSTELLUNG VON ALKALIMETHOXIDEN

PROCÉDÉ INTÉGRÉ DE LA PRODUCTION PARALLÈLE DE MÉTHYLATES DE MÉTAL ALCALIN

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.12.2020 EP 20211938**

(43) Date of publication of application:
**11.10.2023 Bulletin 2023/41**

(73) Proprietor: **BASF SE**
**67056 Ludwigshafen am Rhein (DE)**

(72) Inventors:
• **WEISSKER, Wolf-Steffen**
**67056 Ludwigshafen am Rhein (DE)**
• **GUTH, Josef**
**67251 Freinsheim (DE)**
• **HOFEN, Kai**
**67056 Ludwigshafen am Rhein (DE)**
• **FRIEDRICH, Holger**
**67056 Ludwigshafen am Rhein (DE)**

(74) Representative: **BASF IP Association**
**BASF SE**
**GBI-C006**
**67056 Ludwigshafen (DE)**

(56) References cited:
**US-A1- 2002 183 566     US-A1- 2008 296 786**

(52) Cooperative Patent Classification (CPC): (Cont.)

C-Sets
**C07C 29/70, C07C 31/30**

**Description**

The present invention relates to a new and highly advantageous process for simultaneously preparing alkali metal methoxides in two or more parallel reactive distillation columns. Further, the present invention relates to a chemical production unit for carrying out this process.

In the prior art, processes are described wherein a mixture comprising an alkali metal alkoxide and methanol is prepared in a reactive distillation column from a methanol stream and an aqueous stream which comprises a dissolved alkali metal hydroxide. According to these processes, the methanol stream fed into the reactive distillation column is prepared by separating methanol from water in a distillation column upstream of the reactive distillation column and using the respectively obtained methanol to the reactive distillation column. In this respect, reference is made, for example, to US 2002/0183566 A1, US 2008/0296786 A1, or WO 2013/168113 A1. However, according to the teaching of these prior art documents, only one specific mixture comprising alkali metal methoxide and methanol can be produced. Consequently, for the simultaneous production of e.g. two different mixtures comprising alkali metal methoxide and methanol, such as two mixtures comprising the same alkali metal methoxide but differing in the alkali metal methoxide concentration or two mixtures differing in the alkali metal, two complete separate plants would be necessary, each of the two plants comprising a distillation column for providing the methanol stream and a reactive distillation column. Alternatively, if only one plant is used, it is necessary to produce a first mixture in a first run, stop the first process, re-adjust the production parameters, and start the production of the second mixture. Both alternatives, however, are economically highly disadvantageous since according to the first alternative which would allow for a simultaneous production of two or more different mixtures comprising alkali metal methoxide and methanol, two or more entire plants would be required, leading e.g. to enormous equipment costs; according to the second alternative, the sequential production of the two different mixtures renders the plant disadvantageous in terms of flexibly reacting to changing market demands.

Therefore, it was an object of the present invention to provide an economically advantageous process for simultaneously preparing two or more mixtures comprising alkali metal hydroxide and methanol. It was a further object of the present invention to provide a process for preparing two or more mixtures comprising alkali metal hydroxide and methanol which allows for an easy adjustment of the amount of the mixtures prepared depending on the respective demand of the market.

Surprisingly, it was found that these objects can be solved by a process according to which a single distillation column is employed for generating a methanol stream which is then used, after a suitable dividing into two or more substreams, as a methanol source for two or more parallel downstream reactive distillation columns in which two or more different mixtures comprising alkali metal methoxide and methanol are simultaneously prepared.

Therefore, the present invention relates to an integrated process for simultaneously preparing n mixtures P(i) comprising alkali metal methoxide and methanol, comprising

> providing n reactive distillation columns K(i);
> providing n aqueous liquid streams H(i), a given stream H(i) comprising a dissolved alkali metal hydroxide A(i)OH, wherein n is an integer with $n \geq 2$ and i=1...n; and
> providing a rectification column D;
> the process further comprising

>> (a) providing a stream G comprising methanol;
>> (b) dividing the stream G into n streams G(i), each stream G(i) having the same composition as G;
>> (c) preparing the one or more alkali metal methoxides comprising

>>> feeding each stream G(i) into the lower part of a respective reactive distillation column K(i), and feeding the aqueous liquid stream H(i) comprising the dissolved alkali metal hydroxide A(i)OH into the upper part of said reactive distillation column K(i); and
>>> subjecting G(i) and H(i) in each K(i) to reactive distillation conditions, obtaining n top streams W(i) comprising methanol and water; and obtaining n bottoms streams P(i) comprising alkali metal methoxide A(i)OMe and methanol;

>> (d) feeding each stream W(i) into the lower part of the rectification column D, and feeding a stream M comprising methanol into the rectification column D; and subjecting the n streams W(i) and M in D to distillation conditions, obtaining the stream G according to (a) as a top stream;

>> wherein the rectification column D is operated at a reflux ratio of at least 0.5:1, wherein the rectification column is operated with top vapor recompression, wherein for realizing the reflux ratio the process comprises

(i) removing, in addition to G, a top stream T(2) from the rectification column D, passing said stream T(2) through a condenser V(4), obtaining a liquid stream and a waste gas stream T(2w);

(ii) removing, in addition to G and T(2), a further top stream T(1) from the rectification column D, passing said stream T(2) through a compressor C(3), passing the thus compressed stream through a reboiler V(6), obtaining a liquid stream, wherein the reboiler V(6) is preferably a reboiler of the rectification column D;

(iii) feeding the liquid streams obtained according to (i) and (ii) into the top of the rectification column D;

or

(i) removing, in addition to G, a top stream T(2) from the rectification column D, passing said stream T(2) through a condenser V(4), obtaining a liquid stream T(2l) and a gas stream T(2g); passing the gas stream T(2g) through a condenser V(5), obtaining a liquid stream T(2gl) and a waste gas stream T(2w); and combining the liquid streams T(2l) and (T2gl), obtaining a combined liquid stream T(2cl);

(ii) removing, in addition to G and T(2), a further top stream T(1) from the rectification column D, passing said stream T(2) through a compressor C(3), passing the thus compressed stream through a reboiler V(6), obtaining a liquid stream, wherein the reboiler V(6) is preferably a reboiler of the rectification column D;

(iii) feeding the combined liquid stream obtained according to (i) and the liquid stream obtained according to (ii) into the top of the rectification column D.

Compared to the teaching of the prior art, the process according to the present invention allows for significantly saving apparatus costs since for all reactive distillation columns, only one rectification column is necessary; yet further, the process according to the present invention allows for very flexibly reacting to market demands in terms of the supply of different mixtures comprising alkali metal methoxide and methanol since the respective amounts of different mixtures can be easily adjusted by suitably dividing the methanol stream obtained from the upstream single distillation column.

It is preferred that, in the process according to the present invention, n is in the range of from 2 to 10, more preferably in the range of from 2 to 5, such as 2, 3, 4 or 5, more preferably 2 or 3, more preferably 2.

As to the alkali metal hydroxide A(i)OH, it is preferred that each alkali metal hydroxide A(i)OH is selected from the group consisting of lithium hydroxide, sodium hydroxide and potassium hydroxide, more preferably from the group consisting of sodium hydroxide and potassium hydroxide. According to the process of the present invention, it is conceivable that at least 2 alkali metal hydroxides A(i)OH are the same; in this case, the present invention allow, for example, to prepare a first mixture P(i) comprising a first alkali metal hydroxides A(i)OH and exhibiting a first alkali metal hydroxide concentration and a second mixture P(i) comprising said first alkali metal hydroxide A(i)OH and exhibiting a second alkali metal hydroxide concentration which is different from the first alkali metal hydroxide concentration; thus, according to this conceivable process, it is possible to prepare 2 mixtures P(i) exhibiting different concentration specifications which may demanded by the market. Preferably, at least 2 of the alkali metal hydroxides A(i)OH are different from each other, wherein more preferably, in particular if n is 2 or 3, preferably 2, all alkali metal hydroxides A(i)OH are different from each other.

A given aqueous liquid stream H(i) comprises an alkali metal hydroxide A(i)OH dissolved in water. It may be preferred that at least one of these streams H(i) may comprise, in addition to water and the dissolved alkali metal hydroxide A(i)OH, a certain amount of methanol. Preferably, a given aqueous liquid stream H(i) essentially consists of an alkali metal hydroxide A(i)OH dissolved in water.

As mentioned above, it is preferred that according to the present invention, it is preferred that 2 mixtures P(i) are simultaneously prepared. Therefore, the present invention preferably relates to an integrated process for simultaneously preparing 2 mixtures P(i), wherein the mixture P(1) comprises A(1)OMe, more preferably sodium methoxide, and methanol and the mixture P(2) comprises A(2)OMe, more preferably potassium methoxide, and methanol, the process comprising

(a) providing a stream G comprising methanol;
(b) dividing the stream G into at least two streams G(1) and G(2), G(1) and G(2) having the same composition as G;
(c.1) preparing A(1)OMe, comprising

(c.1.1) feeding the stream G(1) into the lower part of a reactive distillation column K(1), and feeding a liquid stream H(1), preferably an aqueous liquid stream H(1), H(1) comprising dissolved A(1)OH, more preferably sodium hydroxide, into the upper part of the reactive distillation column K(1);

(c.1.2) subjecting G(1) and H(1) in K(1) to reactive distillation conditions, obtaining a top stream W(1) comprising methanol and water; and obtaining a bottoms stream P(1) comprising A(1)OMe and methanol;

(c.2) preparing A(2)OMe, comprising

(c.2.1) feeding the stream G(2) into the lower part of a reactive distillation column K(2), K(2) being arranged in parallel with K(1), and feeding a liquid stream H(2), preferably an aqueous liquid stream H(2), H(2) comprising dissolved A(2)OH, more preferably potassium hydroxide, into the upper part of the reactive distillation column K(2);

(c.2.2) subjecting G(2) and H(2) in K(2) to reactive distillation conditions, obtaining a top stream W(2) comprising methanol and water; and obtaining a bottoms stream P(2) comprising A(2)OMe and methanol;

(d.1) feeding W(1) and W(2) into the lower part of a rectification column D, and feeding a stream M comprising methanol into the rectification column D;

(d.2) subjecting W(1), W(2) and M in D to distillation conditions, obtaining the stream G according to (a) as a top stream.

Preferably, the streams W(1) and W(2) are fed as gas streams into the rectification column D. Generally, these streams can be fed into the rectification column D, independently from each other, at any suitable position. Preferably, these streams are fed, independently from each other, at a position between the bottoms and the 15th theoretical stage, more preferably between the bottoms and the 10th theoretical stage, more preferably between the bottoms and the 8th theoretical stage of the rectification column D. According to the present invention, it is possible to feed the streams W(1) and W(2) into D as separate streams, or to suitably combine the streams W(1) and W(2) and feed the respective combined stream W into D.

As to the rectification column D, it is preferred that it has from 20 to 100, more preferably from 30 to 80, more preferably from 40 to 60 theoretical stages. Conceivable preferred ranges are, for example, from 40 to 50 or from 45 to 55 or from 50 to 60. Generally, the pressure at the top of the rectification column D can be chosen freely within a wide range with the proviso that the desired separation task is fulfilled. Preferably, the rectification column D is operated at a pressure at the top of D in the range of from 0.5 to 10 bar(abs), more preferably in the range of from 0.75 to 6 bar(abs), more preferably in the range of from 1 to 5 bar(abs). Conceivable preferred ranges are, for example, from 1 to 3 bar(abs) or from 2 to 4 bar(abs) or from 3 to 5 bar(abs).

According to the present invention, a stream M comprising methanol is fed into the distillation column D. This stream M, also referred to as fresh methanol stream M, is fed into D in order provide sufficient methanol for the overall process, in particular to compensate the loss of methanol removed from the process via the mixtures P(i). Generally, there are no specific requirements as far as the methanol content of M is concerned, and the skilled person will be in the position to choose suitable methanol streams M. Preferably, however, it is preferred that the stream M comprises only a low amount of water. Therefore, it is further preferred that from 99 to 100 weight-%, more preferably from 99.5 to 100 weight-%, more preferably from 99.9 to 100 weight-% of the stream M consist of methanol and optionally water, wherein the amount of water comprised in the stream M is preferably at most 2000 weight-ppm, more preferably at most 1500 weight-ppm, more preferably at most 1000 weight-ppm, such as at most 750 weight-ppm or at most 500 weight-ppm or at most 250 weight-ppm.

Generally, the stream M can be fed into the rectification column D at any suitable position. Preferably, the stream M is fed to the upper part of D, more preferably at least 2, 3 or 4 theoretical stages from the top of D, more preferably at least 4 theoretical stages from the top of D, more preferably between the 4th and the 20th theoretical stage from the top of D, more preferably between the 6th and the 15th theoretical stage from the top of D, such as between the 6th and the 10th theoretical stage or between to 8th and the 12th theoretical stage or between the 10th and the 14th theoretical stage or between to 12th and the 15th theoretical stage. As far as the temperature of the stream M is concerned at which the stream M is fed into D, it is preferred that the temperature is in the range of from ambient temperature up to the boiling point of methanol at the column pressure of D; more preferably the temperature is ambient temperature.

The rectification column D is operated with reflux. The rectification column D is operated at a reflux ratio of at least 0.5:1, preferably in the range of from 0.55:1 to 1.4:1, more preferably in the range of from 0.6:1 to 1.4:1. Suitable preferred ranges are, for example, from 0.6:1 to 1.0:1 or from 0.8:1 to 1.2:1 or from 1.0:1 to 1.4:1.

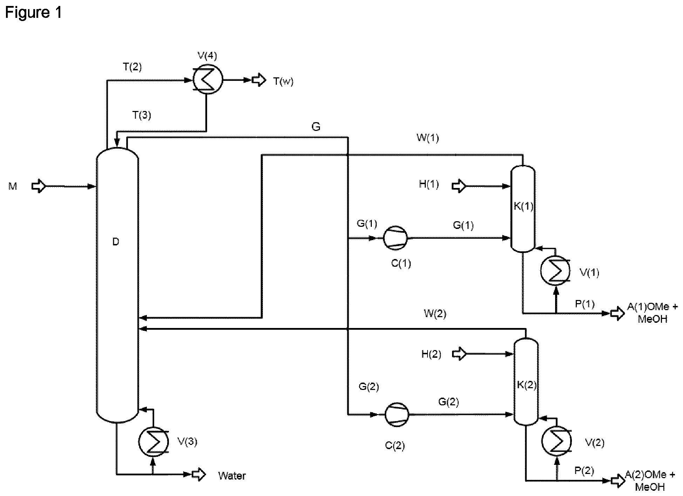

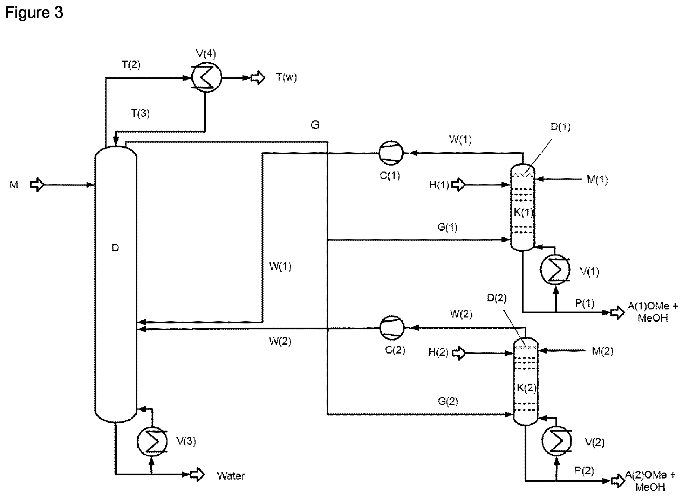

Generally, it is possible that the rectification column D is operated without top vapor recompression - said operation without top vapor recompression not being according to the invention. This is, for example, illustrated by the schematic overview in Figures 1, 2 and 3 showing a process with reflux. In case the rectification column D would be operated without top vapor recompression, it would be possible that realizing the desired reflux ratio comprises removing, in addition to G, a top stream T(2) from the rectification column, passing said stream T(2) through a condenser V(4), obtaining a liquid stream T(3) and a waste gas stream T(2w), and feeding the liquid stream T(3) back into the top of the rectification column D; as far as the process feature "removing, in addition to G, a top stream T(2) from the rectification column" is concerned, it covers the possibility to remove 2 separate streams G and T(2) from the top of D as well as removing one single stream from the top of D and divide said single stream into the 2 streams G and T(2). Alternatively, in case the rectification column D would be operated without top vapor recompression, realizing the desired reflux ratio could comprise removing, in addition to G, a top stream T(2) from the rectification column, passing said stream T(2) through a condenser V(4), obtaining a liquid stream T(2l) and a gas stream T(2g); passing the gas stream T(2g) through a condenser V(5), obtaining a liquid stream T(2gl) and a waste gas stream T(2w); and combining the liquid streams T(2l) and (T2gl), for example in a condensate drum, obtaining a combined liquid stream which is then fed as the stream T(3) back into the top of the rectification column D; again, as far as the process feature "removing, in addition to G, a top stream T(2) from the rectification column" is concerned, it covers the possibility to remove 2 separate streams G and T(2) from the top of D as well as removing one single stream from the top of D and divide said single stream into the 2 streams G and T(2). Certainly, the skilled person may also realize, if need be, said reflux ratio by using more than the 2 condensers V(4) and V(5) mentioned above. As far as the waste gas stream T(2w) is concerned, it is preferred that it essentially consists of oxygen, nitrogen, carbon dioxide and methanol, wherein the amount of methanol in T(2w) is preferably in the range of from 2 to 80 weight-%, more preferably in the range of from 10 to 30 weight-% based on the total weight of T(2w).

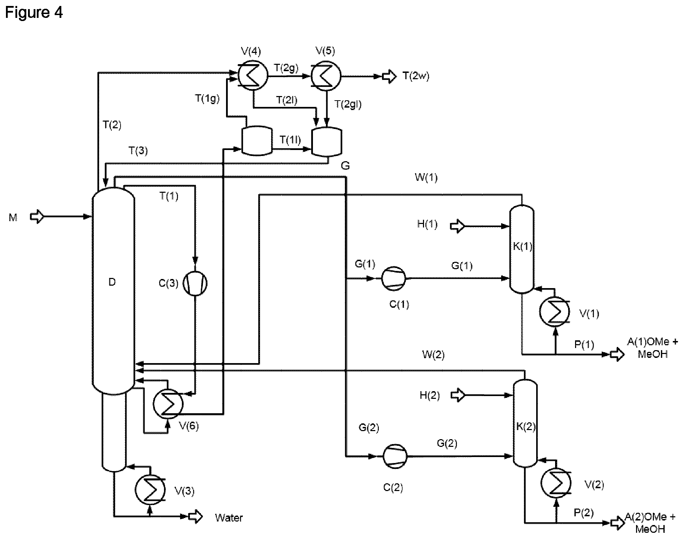

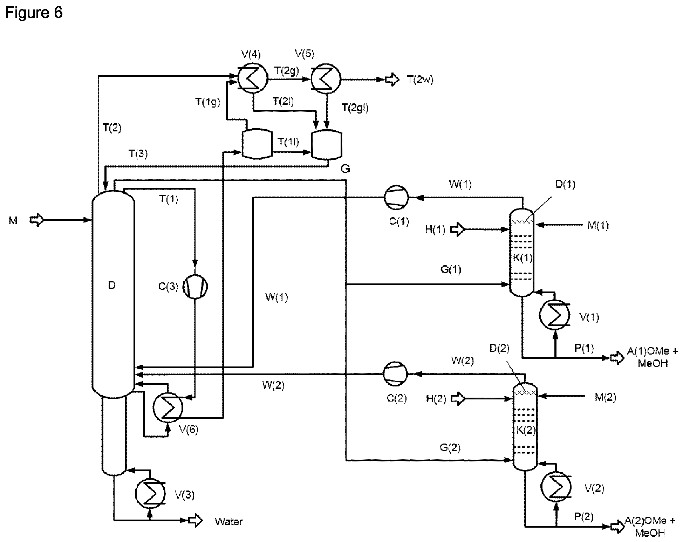

According to the present invention and in particular in view of overall energy consumption topics, the rectification column D is operated with top vapor recompression. Reference is made, for example, to the schematic overview in Figures 4, 5 and 6 showing a process according to the present invention with reflux. As the rectification column is operated with top vapor recompression, realizing the reflux ratio comprises, as indicated above, in one alternative

(i) removing, in addition to G, a top stream T(2) from the rectification column D, passing said stream T(2) through a condenser V(4), obtaining a liquid stream and a waste gas stream T(2w); as far as the process feature "removing, in addition to G, a top stream T(2) from the rectification column" is concerned, it covers the possibility to remove 2 separate streams G and T(2) from the top of D as well as removing one single stream from the top of D and divide said single stream into the 2 streams G and T(2);

(ii) removing, in addition to G and T(2), a further top stream T(1) from the rectification column D, passing said stream T(2) through a compressor C(3), passing the thus compressed stream through a reboiler V(6), obtaining a liquid stream, wherein the reboiler V(6) is preferably a reboiler of the rectification column D; as far as the process feature "removing, in addition to G and T(2), a further top stream T(1) from the rectification column", is concerned, it covers the possibility to remove 3 separate streams G and T(2) from the top of D as well as removing one single stream from the top of D and divide said single stream into the 3 streams G and T(2) and T(1) as well as removing 2 separate streams from the top of D and suitably divide these 2 streams into the streams G, T(2) and T(1);

(iii) feeding the liquid streams obtained according to (i) and (ii) into the top of the rectification column D.

Certainly, as far as step (i) above is concerned, the skilled person may also realize, if need be, said reflux ratio by using more than one condenser V(4) mentioned above. A realization of the use of 2 condensers, V(4) and V(5), is described hereinunder.

As the rectification column is operated with top vapor recompression, realizing the reflux ratio comprises, as indicated above, in another alternative

(i) removing, in addition to G, a top stream T(2) from the rectification column D, passing said stream T(2) through a condenser V(4), obtaining a liquid stream T(2l) and a gas stream T(2g); passing the gas stream T(2g) through a condenser V(5), obtaining a liquid stream T(2gl) and a waste gas stream T(2w); and combining the liquid streams T(2l) and (T2gl), for example in a condensate drum, obtaining a combined liquid stream T(2cl); as far as the process feature "removing, in addition to G, a top stream T(2) from the rectification column" is concerned, it covers the possibility to remove 2 separate streams G and T(2) from the top of D as well as removing one single stream from the top of D and divide said single stream into the 2 streams G and T(2);

(ii) removing, in addition to G and T(2), a further top stream T(1) from the rectification column D, passing said stream T(2) through a compressor C(3), passing the thus compressed stream through a reboiler V(6), obtaining a liquid stream, wherein the reboiler V(6) is preferably a reboiler of the rectification column D; as far as the process feature "removing, in addition to G and T(2), a further top stream T(1) from the rectification column", is concerned, it covers the possibility to remove 3 separate streams G and T(2) from the top of D as well as removing one single stream from the top of D and divide said single stream into the 3 streams G and T(2) and T(1) as well as removing 2 separate streams from the top of D and suitably divide these 2 streams into the streams G, T(2) and T(1); according to process of the invention it may be preferred that the liquid stream obtained from the reboiler V(6) is first fed to a condensate from which a gas stream T(1g) and a liquid stream T(1l) are removed, wherein the gas stream T(1g) may contain, for example, one or more inerts; this stream T(1g) can be fed, for example, into the condenser (V4) or V(5), preferably V(4), wherein the liquid stream T(1l) is preferably combined with the the liquid streams T(2l) and (T2gl), for example in a condensate drum, preferably in the condensate drum described in step (i) above; reference is made to step (iii) below;

(iii) feeding the combined liquid stream obtained according to (i) and the liquid stream obtained according to (ii) into the top of the rectification column D.

Certainly, as far as step (i) above is concerned, the skilled person may also realize, if need be, said reflux ratio by using more than the 2 condensers V(4) and V(5).

As mentioned above, it is preferred that (ii) further comprises feeding the liquid stream obtained from the reboiler V(6) to a condensate drum, wherein from said condensate drum, a gas stream T(1g) and a liquid stream T(1l) are removed, said gas stream T(1g) being fed into the condenser V(4) and said liquid stream T(1l) the liquid stream obtained according to (ii), wherein prior to being fed into the top of the rectification column D according to (iii), the liquid stream is more preferably depressurized. Preferably, (iii) comprises combining the liquid streams obtained according to (i) and (ii) to obtain a liquid stream T(3) and feeding the stream T(3) into the top of the rectification column D.

As far as the waste gas stream T(2w) is concerned, it is preferred that it essentially consists of oxygen, nitrogen, carbon dioxide and methanol, wherein the amount of methanol in T(2w) is preferably in the range of from 2 to 80 weight-%, more preferably in the range of from 10 to 30 weight-% based on the total weight of T(2w).

According to (ii), it is preferred that the reboiler V(6) is an intermediate reboiler of the rectification column D as indicated, for example, in figures 4, 5 and 6. Generally, it would also be conceivable that the reboiler V(6) downstream the compressor C(3) is not used as intermediate reboiler but arranged so as to provide heat to the bottom of the column D. In this case, it would be advantageous to equip the column D with a further bottom reboiler instead of V(3), or together with a smaller dimensioned reboiler V(3), wherein said additional reboiler preferably would be dimensioned smaller than V(3) or V(6), and wherein this further reboiler would be used mainly for starting up the rectification column D. During regular operation mode of the column D, it would be conceivable to switch off said additional reboiler.

With respect to the stream G provided according to (a) obtained from distillation according to (d.2), it is preferred that said stream G comprises methanol and water, wherein more preferably from 99.95 to 100 weight-% of G consist of methanol and water, and wherein the water content of G is at most 200 weight-ppm, more preferably at most 150 weight-ppm, more preferably at most 100 weight-ppm, wherein more preferably, said water content is in the range of from 5 to 100 weight-ppm, more preferably in the range of from 10 to 100 weight-ppm, more preferably in the range of from 15 to 100 weight-ppm.

According to (b), it is preferred that the stream G is divided into the two streams G(1) and G(2), wherein the stream G has a mass flow rate f(G), the stream G(1) has a mass flow rate f(G(1)) and the stream G(2) has a mass flow rate f(G(2)), wherein f(G) = f(G(1)) + f(G(2)). Generally, the stream G can be divided by any conceivable method. Preferably dividing according to (b) comprises passing the stream G into a stream dividing device S, said device more preferably comprising a pipe junction. In context, it is noted that the term "the stream is divided into two streams" refers to a method according to which the streams obtained from said dividing have the same chemical composition as the stream G. As far as the ratios f(G(1))/f(G) and f(G(2))/f(G) are concerned, the present invention allows for a flexible adjusting of said ratios in that the individual flow rates f(G(1)) and f(G(2)) can be chosen depending on the desired amount of A(1)OMe, preferably sodium methoxide, to be obtained according to (c.1.2) relative to the desired amount of A(2)OMe, preferably potassium methoxide, to be obtained according to (c.2.2).

Prior to dividing according to (b), the stream G can be passed through a compressor C, thereby realizing a pressure increase of G. Preferably, the pressure is suitably increased so that the pressure of the streams after dividing is adapted to the desired pressure when the streams are fed into the reactive distillation columns K(i) and ultimately, via the streams W(i), back into D. Preferably, said pressure increase is in the range of from 0.1 to 0.8 bar(abs), more preferably in the range of from 0.15 to 0.6 bar(abs), more preferably in the range of from 0.2 to 0.4 bar(abs). According to this embodiment of the present invention, it is preferred that the dividing according to (b) comprises passing the compressed stream G into a stream dividing device S, said device preferably comprising a pipe junction and at least one control device allowing for adjusting the ratio f(G(1))/f(G(2)), wherein said at least one control device is located downstream of said pipe junction. At least one of these control devices is located either in the stream G(1) or in the stream G(2) or in both streams G(1) and G(2), and it is preferred that the at least one control device preferably a control valve.

Preferably, according to the present invention, the pressure increase mentioned above is realized not by compressing the stream G prior to, but after dividing. Thus, according to the present invention, it is preferred that prior to be fed into the reactive distillation column K(1), the stream G(1) is passed through a compressor C(1), thereby realizing a pressure increase of G(1) in the range of from 0.1 to 0.8 bar(abs), preferably in the range of from 0.15 to 0.6 bar(abs), more preferably in the range of from 0.2 to 0.4 bar(abs). Certainly, said compression of G(1) can be combined with a pre-compression of the stream G prior to dividing; however, it is preferred that this compressing of G(1) is performed with no compression of G being performed prior to dividing according to (b). Consequently, it is also preferred that prior to be fed into the reactive distillation column K(2), the stream G(2) is passed through a compressor C(2), thereby realizing a pressure increase of G(2) in the range of from 0.1 to 0.8 bar(abs), preferably in the range of from 0.15 to 0.6 bar(abs), more preferably in the range of from 0.2 to 0.4 bar(abs). Certainly, said compression of G(2) can be combined with a pre-compression of the stream G prior to dividing; however, it is preferred that this compressing of G(2) is performed with no compression of G being performed prior to dividing according to (b).

As far as the stream H(1) is concerned, it is preferred that from 99 to 100 weight-%, more preferably from 99.5 to 100 weight-%, more preferably from 99.9 to 100 weight-% of the stream H(1) consist of A(1)OH and water, wherein more preferably from 37.5 to 58 weight-%, more preferably from 40 to 55 weight-%, more preferably from 42.5 to 52 weight-% of the stream H(1) consist of A(1)OH, preferably sodium hydroxide. Preferably the stream H(1) is fed into the reactive distillation column K(1) at a temperature of H(1) in the range of from ambient temperature to its boiling temperature, more preferably in the range of from 50 to 80 °C such as from 50 to 60 °C or from 60 to 70 °C or from 70 to 80 °C. Heating of the stream H(1) to this temperature may be accomplished with any suitable means such as a heat exchanger. It is preferred that the stream H(1) is fed into the top of the reactive distillation column K(1), more preferably to the first theoretical stage from the top.

As to the reactive distillation column K(1), it is preferred that said column has from 5 to 50, more preferably from 10 to 40, more preferably from 15 to 30 theoretical stages, such as from 15 to 20 or from 20 to 25 or from 25 to 30 theoretical stages. Generally, the stream G(1) can be fed at any suitable position into K(1); preferably, G(1) is fed into the reactive distillation column K(1) at a position between the bottoms and the 5th theoretical stage, more preferably between the bottoms and the 3rd theoretical stage, more preferably between the bottoms and the 2nd theoretical stage of the reactive distillation column K(1). Preferably, the reactive distillation column K(1) is operated at a pressure at the top in the range of from 0.5 to 10 bar(abs), more preferably in the range of from 1 to 6 bar(abs), more preferably in the range of from 1 to 5 bar(abs). Suitable preferred ranges are, for example, from 1 to 3 bar(abs) or from 2 to 4 bar(abs) of from 3 to 5 bar(abs). While it is generally possible to operate the reactive distillation column K(1) with reflux, it is preferred that the reactive distillation column K(1) is operated at a reflux ratio of 0:1.

As far as the stream W(1) is concerned which is obtained from the top of K(1), it is preferred that from 99 to 100 weight-%, more preferably from 99.5 to 100 weight-%, more preferably from 99.9 to 100 weight-% of W(1) consist of methanol and water. More preferably, from 1 to 10 weight-%, more preferably from 2 to 8 weight-%, more preferably from 4 to 7 weight-%, more preferably from 5 to 6 weight-% of the stream W(1) consist of water.

As far as the mixture P(1) is concerned, it is preferred that from 99 to 100 weight-%, more preferably from 99.5 to 100 weight-%, more preferably from 99.9 to 100 weight-% of the stream P(1) consist of A(1)OMe, preferably sodium methoxide, and methanol. More preferably, from 10 to 50 weight-%, more preferably from 20 to 40 weight-%, more preferably from 25 to 35 weight-% of the stream P(1) consist of A(1)OMe, preferably sodium methoxide. More preferably, at most 5000 weight-ppm, more preferably at most 2000 weight-ppm, more preferably at most 1000 weight-ppm of the stream P(1) consist of water. Conceivable maximum water contents may include, for example, 750 weight-ppm or 500 weight-ppm or 250 weight-ppm. Preferably, the concentration of A(1)OMe, preferably sodium methoxide in the stream P(1) are realized by the skilled person by operating the reactive distillation column K(1) at a respective reboiler duty.

According to the present invention, it is preferred that the top of the reactive distillation column K(1) is equipped with a suitable droplet separating device D(1), preferably a demister. Thus, the process preferably comprises separating droplets comprising A(1)OH, preferably sodium hydroxide, from the vapor stream in the top of K(1). It is further preferred that, in particular for cleaning purposes, said demister is suitably treated with a suitable stream M(1). A preferred treating may comprise, preferably consist of at least temporarily spraying the demister with the stream M(1). Regarding the chemical composition of M(1), it is especially preferred that M(1) comprises methanol, wherein it is more preferred that M(1) is branched from a condensed top stream removed from the rectification column D, for example one of the streams described above, or being a fresh methanol stream, for example a stream branched from the stream M described above.

As far as the stream H(2) is concerned, it is preferred that from 99 to 100 weight-%, more preferably from 99.5 to 100 weight-%, more preferably from 99.9 to 100 weight-% of the stream H(2) consist of A(2)OH, preferably potassium hydroxide, and water, wherein more preferably 30 to 55 weight-%, more preferably from 40 to 52.5 weight-%, more preferably from 45 to 50 weight-% of the stream H(2) consist of A(2)OH, preferably potassium hydroxide. Preferably the stream H(2) is fed into the reactive distillation column K(2) at a temperature of H(2) in the range of from ambient temperature to its boiling temperature, more preferably in the range of from 50 to 80 °C such as from 50 to 60 °C or from 60 to 70 °C or from 70 to 80 °C. Heating of the stream H(2) to this temperature may be accomplished with any suitable means such as a heat exchanger. It is preferred that the stream H(2) is fed into the top of the reactive distillation column K(2), more preferably to the first theoretical stage from the top.

As to the reactive distillation column K(2), it is preferred that said column has from 5 to 50, more preferably from 10 to 40, more preferably from 15 to 30 theoretical stages, such as from 15 to 20 or from 20 to 25 or from 25 to 30 theoretical stages. Generally, the stream G(2) can be fed at any suitable position into K(2); preferably, G(2) is fed into the reactive distillation column K(2) at a position between the bottoms and the 5th theoretical stage, more preferably between the bottoms and the 3rd theoretical stage, more preferably between the bottoms and the 2nd theoretical stage of the reactive distillation column K(2). Preferably, the reactive distillation column K(2) is operated at a pressure at the top in the range of from 0.5 to 10 bar(abs), more preferably in the range of from 1 to 6 bar(abs), more preferably in the range of from 1 to 5 bar(abs). Suitable preferred ranges are, for example, from 1 to 3 bar(abs) or from 2 to 4 bar(abs) of from 3 to 5 bar(abs). While it is generally possible to operate the reactive distillation column K(2) with reflux, it is preferred that the reactive distillation column K(2) is operated at a reflux ratio of 0:1.

As far as the stream W(2) is concerned which is obtained from the top of K(2), it is preferred that from 99 to 100 weight-%, more preferably from 99.5 to 100 weight-%, more preferably from 99.9 to 100 weight-% of W(2) consist of methanol and water. More preferably, from 1 to 15 weight-%, more preferably from 2 to 12 weight-%, more preferably from 6 to 10 weight-% of the stream W(2) consist of water.

As far as the mixture P(2) is concerned, it is preferred that from 99 to 100 weight-%, more preferably from 99.5 to 100 weight-%, more preferably from 99.9 to 100 weight-% of the stream P(2) consist of A(2)OMe, preferably potassium methoxide, and methanol. More preferably, from 10 to 50 weight-%, more preferably from 20 to 40 weight-%, more preferably from 25 to 35 weight-% of the stream P(2) consist of A(2)OMe, preferably potassium methoxide. More preferably, at most 5000 weight-ppm, more preferably at most 2000 weight-ppm, more preferably at most 1000 weight-ppm of the stream P(2) consist of water. Conceivable maximum water contents may include, for example, 750 weight-ppm or 500 weight-ppm or 250 weight-ppm. Preferably, the concentration of A(2)OM, preferably potassium methoxide in the stream P(2) are realized by the skilled person by operating the reactive distillation column K(2) at a respective reboiler duty.

According to the present invention, it is preferred that the top of the reactive distillation column K(2) is equipped with a suitable droplet separating device D(2), preferably a demister. Thus, the process preferably comprises separating droplets comprising A(2)OH, preferably potassium hydroxide, from the vapor stream in the top of K(2). It is further preferred that, in particular for cleaning purposes, said demister is suitably treated with a suitable stream M(2). A preferred treating may comprise, preferably consist of at least temporarily spraying the demister with the stream M(2). Regarding the chemical composition of M(2), it is especially preferred that M(2) comprises methanol, wherein it is more preferred that M(2) is branched from a condensed top stream removed from the rectification column D, for example one of the streams described above, or being a fresh methanol stream, for example a stream branched from the stream M described above.

In the above, it was described that according to the present invention, either the stream G, prior to dividing, is passed through a compressor C, and/or after dividing, the stream G(1) is passed through a compressor C(1) and the stream G(2) is passed through a compressor C(2), the latter being preferred. According to the present invention, it is also possible that either in addition to at least one of the above alternatives or, preferably as the sole respective compression, prior to being fed into the rectification column D, the stream W(1) is passed through a compressor C(1), thereby realizing a pressure increase of W(1) preferably in the range of from 0.1 to 0.8 bar(abs), more preferably in the range of from 0.15 to 0.6 bar(abs), more preferably in the range of from 0.2 to 0.4 bar(abs), and prior to being fed into the rectification column D, the stream W(2) is passed through a compressor C(2), thereby realizing a pressure increase of W(2) in the range of from 0.1 to 0.8 bar(abs), more preferably in the range of from 0.15 to 0.6 bar(abs), more preferably in the range of from 0.2 to 0.4 bar(abs). According to this embodiment of the present invention, it is also possible to suitably combine the streams W(1) and W(2), prior to being passed through a compressor, in a combining device to obtain a respective combined stream W, and pass said combined stream W, prior to being fed into D, through a compressor, thereby realizing a pressure increase of W(1) preferably in the range of from 0.1 to 0.8 bar(abs), more preferably in the range of from 0.15 to 0.6 bar(abs), more preferably in the range of from 0.2 to 0.4 bar(abs). Said combining device preferably comprises a pipe junction and at least one control device, preferably a control valve.

As far as the integrated process of the present invention is concerned, it is noted that for simultaneously preparing, in addition to the 2 mixtures P(1) and P(2) as described above in detail, a 3rd mixture P(3), and/or a 4th mixture P(4), and/or a 5th mixture P(5) etc., the skilled person, based on his general knowledge, will be in the position to derive from said details above in a straight-forward manner also any detail for operating the respective additional compressors, reactive distillation columns, and the like. In particular, according to the present invention, it is preferred that the process of the present invention is an integrated process for simultaneously preparing three mixtures P(1), P(2) and P(3), wherein the mixture P(1) comprises sodium methoxide and methanol, the mixture P(2) comprises potassium methoxide and methanol, and the mixture P(3) comprises lithium methoxide and methanol, the process comprising

(a) providing a stream G comprising methanol;
(b) dividing the stream G into three streams G(1), G(2) and G(3), G(1), G(2) and G(3) having the same composition as G;
(c.1) preparing sodium methoxide comprising

(c.1.1) feeding the stream G(1) into the lower part of a reactive distillation column K(1), and feeding an aqueous liquid stream H(1) comprising dissolved sodium hydroxide, into the upper part of the reactive distillation column K(1);
(c.1.2) subjecting G(1) and H(1) in K(1) to reactive distillation conditions, obtaining a top stream W(1) comprising methanol and water; and obtaining a bottoms stream P(1) comprising sodium methoxide and methanol;

(c.2) preparing potassium methoxide comprising

(c.2.1) feeding the stream G(2) into the lower part of a reactive distillation column K(2), K(2) being arranged in parallel with K(1), and feeding an aqueous liquid stream H(2) comprising dissolved potassium hydroxide, into the upper part of the reactive distillation column K(2);

(c.2.2) subjecting G(2) and H(2) in K(2) to reactive distillation conditions, obtaining a top stream W(2) comprising methanol and water; and obtaining a bottoms stream P(2) comprising potassium methoxide and methanol;

(c.3) preparing lithium methoxide comprising

(c.3.1) feeding the stream G(3) into the lower part of a reactive distillation column K(3), K(3) being arranged in parallel with K(1) and K(2), and feeding an aqueous liquid stream H(3) comprising dissolved lithium hydroxide, into the upper part of the reactive distillation column K(3);

(c.3.2) subjecting G(3) and H(3) in (K3) to reactive distillation conditions, obtaining a top stream W(3) comprising methanol and water; and obtaining a bottoms stream P(3) comprising lithium methoxide and methanol;

(d.1) feeding W(1), W(2) and W(3) into the lower part of a rectification column D, and feeding a stream M comprising methanol into the rectification column D;

(d.2) subjecting W(1), W(2), W(3) and M in D to distillation conditions, obtaining the stream G according to (a) as a top stream.

In particular as far as the steps (c.3) including (c.3.1) and (c.3.2), step (d.1) as far as W(3) is concerned and step (d.2) as far as W(3) is concerned, the skilled person, based on his general knowledge, will be in the position to derive from said details above in a straight-forward manner also any detail for operating the respective additional compressors, such as C(3) analogously to C(1) and C(2), the addition reactive distillation columns K(3) including, for example the additional droplet separator device, preferably the demister, and the preferred additional stream M(3) analogously to M(1) and M(2), and the like. At least two of the streams W(1), W(2) and W(3) may be suitably combined prior to being fed into D.

According to the present invention, it is also conceivable that from at least one of the streams P(i), A(i)OMe is at least partially separated from methanol, more preferably obtaining solid, more preferably crystalline A(i)OMe. Thus, solid, preferably crystalline sodium methoxide and solid, preferably crystalline potassium methoxide, can be obtained. According to the above-described process wherein 2 mixtures P(i) are prepared, it would also be preferred that, in addition, solid, preferably crystalline lithium methoxide can be obtained.

The present invention further relates to a chemical production unit for carrying out the process according to the present invention, comprising

- a rectification column D comprising

-- inlet means for feeding the stream M into D, preferably in the upper part of D;
-- in its lower part, inlet means for feeding the streams W(i) or one or more combined stream thereof into D;
-- outlet means for removing the streams T(2) and G or a combined stream thereof from the top of D;
-- at least one condenser, preferably a condenser V(4) and optionally a further condenser V(5) arranged downstream of V(4), having inlet means for receiving the stream T(2) and having outlet means for removing the condensed stream T(3) and for removing a waste gas stream;
-- inlet means for feeding the stream T(3) to the top of D;
-- a bottom reboiler;

- a stream dividing device S for dividing the stream G into n streams G(i);
- means for passing the stream G to said stream dividing device S;
- n reactive distillation columns K(i), n≥2 and i=1...n; each reactive distillation column K(i) comprising

-- in its upper part, preferably in its top, inlet means for feeding a stream H(i) into K(i);
-- in its lower part, inlet means for feeding a stream G(i) into K(i);
-- outlet means for removing a stream W(i) from the top of K(i);
-- a bottom reboiler;
-- outlet means for removing a bottoms stream from K(i);
-- a stream dividing means for separating a stream P(i) from the bottoms stream removed from K(i);

- means for passing the streams G(i) to the reactive distillation columns K(i);
- means for passing the streams W(i) to the rectification column D;
- one or more compressors C(i) for compressing either the stream G and/or the streams G(i) and/or the streams W(i);

the unit further comprising means for realizing recompression of the top vapor obtained from D, said means comprising a compressor C(3) for compressing a stream T(1) removed from the top of D, means for passing T(1) from the top of D to C(3), a reboiler V(6) for condensing said compressed stream, means for passing said compressed stream from (C3) to V(6), and means for feeding the obtained liquid stream to the top of D, wherein the reboiler V(6) is an intermediate reboiler of D.

Preferably, the top of at least one, more preferably of each reactive distillation column K(i) is equipped with a droplet separating device D(i), more preferably a demister, said demister more preferably comprising an inlet means for feeding a stream M(i) comprising methanol into said demister. Preferably each of the reactive distillations columns K(i) comprises, independently from one another, from 5 to 50, more preferably from 10 to 40, more preferably from 15 to 30 theoretical stages. It is preferred that the means for passing the streams G(i) to the reactive distillation columns K(i) are located, independently from one another, at a position between the bottoms and the fifth theoretical stage, more preferably between the bottoms and the third theoretical stage, more preferably between the bottoms and the second theoretical stage of K(i). Preferably, the means for passing the streams H(i) into the reactive distillation columns K(i) are located at the top of K(i), preferably at the uppermost theoretical stage. Preferably at least one, more preferably each reactive distillation columns K(i) does not comprise means for being operated at a reflux ratio of greater than 0:1. Preferably each reactive distillation column K(i) is equipped with trays.

It is preferred that the unit of the present invention comprises n compressors C(i) arranged upstream of K(i) for compressing the streams G(i). Alternatively, it is preferred that the unit of the present invention comprises n compressors C(i) arranged downstream of K(i) and upstream of D for compressing the streams W(i).

As to the rectification column D, it is preferred that said column has from 20 to 100, more preferably from 30 to 80, more preferably from 40 to 60 theoretical stages. Preferably, the inlet means of D for feeding the streams W(i) or one or more combined stream thereof into D is/are located at a position between the bottoms and the $15^{th}$ theoretical stage, more preferably between the bottoms and the $10^{th}$ theoretical stage, more preferably between the bottoms and the $8^{th}$ theoretical of D. It is preferred that the inlet means for feeding the stream M into D are located at least 4 theoretical stages from the top of D, more preferably between the $4^{th}$ and the $20^{th}$ theoretical stage from the top of D, more preferably between the $6^{th}$ and the $15^{th}$ theoretical stage from the top of D.

It is preferred that the unit of the present invention further comprises at least one condensate drum for a liquid stream removed from V(4) and optionally from V(5) and further comprising means for passing at least part of the liquid contained in said drum as the stream T(3) to the top of D. It is further preferred that the rectification column D is equipped with trays and/or packings, wherein, insofar as the reboiler V(6) is a reboiler of D, D is more preferably equipped with packings arranged above the intermediate reboiler of D and with trays arranged below the intermediate reboiler of D.

In the context of the present invention, it is also conceivable that suitable reactive distillation columns K(i) are essentially bubble cap tray, valve tray and sieve tray columns. Specifically in the case of valve trays and sieve trays, the trays should be configured so that the raining-through of the liquid is minimized. A person skilled in the art will be familiar with the constructional measures required for this. Particularly tightly closing valve types are selected and thus, in particular, the vapor velocity into the tray openings is increased to double the value which is customarily set. This is achieved by a reduction in the number of valves. In the case of sieve trays, it is particularly useful to reduce the diameter of the openings in the tray and to maintain or even increase the number of openings. It is also conceivable that the columns are provided with random packing elements or ordered packing, with ordered packing being preferred over random packing elements with a view to uniform distribution of the liquid. The average ratio of liquid flow to vapor flow must not be exceeded by more than 15%, preferably not by more than 3%, in all sub-regions of the column cross section which correspond to more than 2% of the total column cross section. This low amount of liquid to be maintained makes it possible for the capillary effect on the wire meshes to prevent local peak values of the liquid trickle density.

According to the present invention, it is preferred that the unit further comprises at least one condensate drum for the condensed stream removed from V(6), the unit more preferably further comprising means for passing at least part of a gas phase in said drum to V(4) and means for passing at least part of a liquid phase in said drum to a condensate drum as defined in the foregoing.

Further, it may be preferred that the unit further comprises means for separating an alkali metal methoxide A(i)OMe from at least one of the streams P(i).

Preferably, the number of reactive distillation columns K(i), n, is in the range of from 2 to 10, more preferably in the range of from 2 to 5, more preferably 2 or 3, more preferably 2.

The present invention further relates to a use of a chemical production unit according to the present invention or of a process according to the present invention for simultaneously producing n mixtures P(i) comprising alkali metal methoxide and methanol, n being an integer with n≥2 and i=1...n, wherein either at least 2 of the mixtures P(i) comprise different alkali metal methoxides A(i)OMe, and/or at least 2 of the mixtures P(i) comprise the same alkali metal alkoxide A(i)OMe at different concentrations.

The present invention is further illustrated by the following set of embodiments and combinations of embodiments resulting from the dependencies and back-references as indicated. In particular, it is noted that in each instance where a range of embodiments is mentioned, for example in the context of a term such as "The process of any one of embodiments 1 to 4", every embodiment in this range is meant to be explicitly disclosed for the skilled person, i.e. the wording of this term is to be understood by the skilled person as being synonymous to "The process of any one of embodiments 1, 2, 3 and 4".

Further, it is explicitly noted that the following set of embodiments is not the set of claims determining the extent of protection, but represents a suitably structured part of the description directed to general and preferred aspects of the present invention.

Embodiment 1: An integrated process for simultaneously preparing n mixtures P(i) comprising alkali metal methoxide and methanol, comprising

> providing n reactive distillation columns K(i);
> providing n aqueous liquid streams H(i), a given stream H(i) comprising a dissolved alkali metal hydroxide A(i)OH, wherein n is an integer with n≥2 and i=1...n; and
> providing a rectification column D;
> the process further comprising

>> (a) providing a stream G comprising methanol;
>> (b) dividing the stream G into n streams G(i), each stream G(i) having the same composition as G;
>> (c) preparing the one or more alkali metal methoxides comprising

>>> feeding each stream G(i) into the lower part of a respective reactive distillation column K(i), and feeding the aqueous liquid stream H(i) comprising the dissolved alkali metal hydroxide A(i)OH into the upper part of said reactive distillation column K(i); and
>>> subjecting G(i) and H(i) in each K(i) to reactive distillation conditions, obtaining n top streams W(i) comprising methanol and water; and obtaining n bottoms streams P(i) comprising alkali metal methoxide A(i)OMe and methanol;

>> (d) feeding each stream W(i) into the lower part of the rectification column D, and feeding a stream M comprising methanol into the rectification column D; and subjecting the n streams W(i) and M in D to distillation conditions, obtaining the stream G according to (a) as a top stream;

> wherein the rectification column D is operated at a reflux ratio of at least 0.5:1, wherein the rectification column is operated with top vapor recompression,
> wherein for realizing the reflux ratio the process comprises

>> (i) removing, in addition to G, a top stream T(2) from the rectification column D, passing said stream T(2) through a condenser V(4), obtaining a liquid stream and a waste gas stream T(2w);
>> (ii) removing, in addition to G and T(2), a further top stream T(1) from the rectification column D, passing said stream T(2) through a compressor C(3), passing the thus compressed stream through a reboiler V(6), obtaining a liquid stream, wherein the reboiler V(6) is preferably a reboiler of the rectification column D;
>> (iii) feeding the liquid streams obtained according to (i) and (ii) into the top of the rectification column D;

> or

>> (i) removing, in addition to G, a top stream T(2) from the rectification column D, passing said stream T(2) through a condenser V(4), obtaining a liquid stream T(2l) and a gas stream T(2g); passing the gas stream T(2g) through a condenser V(5), obtaining a liquid stream T(2gl) and a waste gas stream T(2w); and combining the liquid streams T(2l) and (T2gl), obtaining a combined liquid stream T(2cl);
>> (ii) removing, in addition to G and T(2), a further top stream T(1) from the rectification column D, passing said stream T(2) through a compressor C(3), passing the thus compressed stream through a reboiler V(6), obtaining a liquid stream, wherein the reboiler V(6) is preferably a reboiler of the rectification column D;
>> (iii) feeding the combined liquid stream obtained according to (i) and the liquid stream obtained according to (ii) into the top of the rectification column D.

Embodiment 2: The process of embodiment 1, wherein n is in the range of from 2 to 10, preferably in the range of from 2 to 5, more preferably 2 or 3, more preferably 2;

Embodiment 3: The process of embodiment 1 or 2, wherein each alkali metal hydroxide A(i)OH is preferably selected from the group consisting of lithium hydroxide, sodium hydroxide and potassium hydroxide, more preferably from the group consisting of sodium hydroxide and potassium hydroxide.

Embodiment 4: The process of any one of embodiments 1 to 3, wherein at least 2 of the alkali metal hydroxides A(i)OH are different from each other, wherein preferably, in particular if n is 2 or 3, all alkali metal hydroxides A(i)OH are different from each other.

Embodiment 5: The process of any one of embodiments 1 to 4, wherein a given liquid stream H(i) comprises an alkali metal hydroxide A(i)OH dissolved in water, in methanol, or in a mixture comprising water and methanol, preferably in water.

Embodiment 6: The process of any one of embodiments 1 to 5, being an integrated process for simultaneously preparing at least 2, preferably 2 mixtures P(i), wherein the mixture P(1) comprises A(1)OMe, preferably sodium methoxide, and methanol and the mixture P(2) comprises A(2)OMe, preferably potassium methoxide, and methanol, the process comprising

(a) providing a stream G comprising methanol;
(b) dividing the stream G into at least two streams G(1) and G(2), G(1) and G(2) having the same composition as G;
(c.1) preparing A(1)OMe, comprising

(c.1.1) feeding the stream G(1) into the lower part of a reactive distillation column K(1), and feeding a liquid stream H(1), preferably an aqueous liquid stream H(1), H(1) comprising dissolved A(1)OH, preferably sodium hydroxide, into the upper part of the reactive distillation column K(1);
(c.1.2) subjecting G(1) and H(1) in K(1) to reactive distillation conditions, obtaining a top stream W(1) comprising methanol and water; and obtaining a bottoms stream P(1) comprising A(1)OMe and methanol;

(c.2) preparing A(2)OMe, comprising

(c.2.1) feeding the stream G(2) into the lower part of a reactive distillation column K(2), K(2) being arranged in parallel with K(1), and feeding a liquid stream H(2), preferably an aqueous liquid stream H(2), H(2) comprising dissolved A(2)OH, preferably potassium hydroxide, into the upper part of the reactive distillation column K(2);
(c.2.2) subjecting G(2) and H(2) in K(2) to reactive distillation conditions, obtaining a top stream W(2) comprising methanol and water; and obtaining a bottoms stream P(2) comprising A(2)OMe and methanol;

(d.1) feeding W(1) and W(2) into the lower part of a rectification column D, and feeding a stream M comprising methanol into the rectification column D;
(d.2) subjecting W(1), W(2) and M in D to distillation conditions, obtaining the stream G according to (a) as a top stream.

Embodiment 7: The process of embodiment 6, wherein W(1) and W(2) are fed as gas streams into the rectification column D, preferably at a position between the bottoms and the 15th theoretical stage, preferably between the bottoms and the 10th theoretical stage, more preferably between the bottoms and the 8th theoretical of the rectification column D.

Embodiment 8: The process of any one of embodiments 1 to 7, wherein the rectification column D has from 20 to 100, preferably from 30 to 80, more preferably from 40 to 60 theoretical stages.

Embodiment 9: The process of any one of embodiments 1 to 8, wherein the rectification column D is operated at a pressure at the top in the range of from 0.5 to 10 bar(abs), preferably in the range of from 0.75 to 6 bar(abs), more preferably in the range of from 1 to 5 bar(abs).

Embodiment 10: The process of any one of embodiments 1 to 9, wherein from 99 to 100 weight-%, preferably from 99.5 to 100 weight-%, more preferably from 99.9 to 100 weight-% of the stream M consist of methanol and optionally water, wherein the amount of water comprised in the stream M is at most 2000 weight-ppm, more preferably at most 1500 weight-ppm, more preferably at most 1000 weight-ppm.

Embodiment 11: The process of any one of embodiments 1 to 10, wherein the stream M is fed to the upper part of D, preferably at least 4 theoretical stages from the top of D, more preferably between the 4th and the 20th theoretical stage from the top of D, more preferably between the 6th and the 15th theoretical stage from the top of D, preferably at a temperature of M in the range of from ambient temperature to the boiling point of methanol at the column pressure of D, more preferably at ambient temperature.

Embodiment 12: The process of any one of embodiments 1 to 11, wherein the rectification column D is operated at a reflux ratio in the range of from 0.55:1 to 1.4:1, more preferably in the range of from 0.6:1 to 1.4:1.

Embodiment 13: The process of any one of embodiments 1 to 12, wherein the waste gas stream T(2w) essentially consists of oxygen, nitrogen, carbon dioxide and methanol, wherein the amount of methanol in T(2w) is preferably in the range of from 2 to 80 weight-%, preferably in the range of from 10 to 30 weight-% based on the total weight of T(2w).

Embodiment 14: The process of any one of embodiments 1 to 13, wherein (ii) further comprises feeding the liquid stream obtained from the reboiler V(6) to a condensate drum, wherein from said condensate drum, a gas stream T(1g) and a liquid stream T(1l) are removed, said gas stream T(1g) being fed into the condenser V(4) and said liquid stream T(1l) the liquid stream obtained according to (ii), wherein prior to being fed into the top of the rectification column D according to (iii), the liquid stream is preferably depressurized.

Embodiment 15: The process of any one of embodiments 1 to 14, wherein (iii) comprises combining the liquid streams obtained according to (i) and (ii) to obtain a liquid stream T(3) and feeding the stream T(3) into the top of the rectification column D.

Embodiment 16: The process of any one of embodiments 1 to 15, wherein according to (ii), the reboiler V(6) is an intermediate reboiler of the rectification column D or the bottom reboiler of the rectification column D, preferably an intermediate reboiler of the rectification column D.

Embodiment 17: The process of any one of embodiments 1 to 16, wherein the stream G provided according to (a) by obtaining from distillation according to (d.2) comprises methanol and water, wherein preferably from 99.95 to 100 weight-% of G consist of methanol and water, and wherein the water content of G is at most 200 weight-ppm, preferably at most 150 weight-ppm, more preferably at most 100 weight-ppm, wherein more preferably said water content is in the range of from 5 to 100 weight-ppm, more preferably in the range of from 10 to 100 weight-ppm, more preferably in the range of from 15 to 100 weight-ppm.

Embodiment 18: The process of any one of embodiments 1 to 17, wherein according to (b), the stream G is divided into the two streams G(1) and G(2), wherein the stream G has a mass flow rate f(G), the stream G(1) has a mass flow rate f(G(1)) and the stream G(2) has a mass flow rate f(G(2)), wherein f(G) = f(G(1)) + f(G(2)).

Embodiment 19: The process of any one of embodiments 1 to 18, wherein dividing according to (b) comprises passing the stream G into a stream dividing device S, said device preferably comprising a pipe junction.

Embodiment 20: The process of embodiment 18 or 19, wherein the ratios f(G(1))/f(G) and f(G(2))/f(G) are adjusted depending on the desired amount of A(1)OMe to be obtained according to (c.1.2) relative to the desired amount of A(2)OMe to be obtained according to (c.2.2).

Embodiment 21: The process of any one of embodiments 1 to 20, wherein prior to dividing according to (b), the stream G is passed through a compressor C, thereby realizing a pressure increase of G in the range of from 0.1 to 0.8 bar(abs), preferably in the range of from 0.15 to 0.6 bar(abs), more preferably in the range of from 0.2 to 0.4 bar(abs).

Embodiment 22: The process of embodiment 21, wherein dividing according to (b) comprises passing the compressed stream G into a stream dividing device S, said device preferably comprising a pipe junction and at least one control device allowing for adjusting the ratio f(G(1))/f(G(2)) as defined in embodiment 25, said at least one control device being located downstream of said pipe junction, wherein at least one of these control devices is located either in the stream G(1) or in the stream G(2) or in both streams G(1) and G(2), wherein said at least one control device preferably comprises a control valve.

Embodiment 23: The process of any one of embodiments 1 to 22, preferably of any one of embodiments 1 to 27, wherein prior to be fed into the reactive distillation column K(1), the stream G(1) is passed through a compressor C(1), thereby realizing a pressure increase of G(1) in the range of from 0.1 to 0.8 bar(abs), preferably in the range of from 0.15 to 0.6 bar(abs), more preferably in the range of from 0.2 to 0.4 bar(abs).

Embodiment 24: The process of any one of embodiments 1 to 23, preferably of any one of embodiments 1 to 27, wherein prior to be fed into the reactive distillation column K(2), the stream G(2) is passed through a compressor C(2), thereby realizing a pressure increase of G(2) in the range of from 0.1 to 0.8 bar(abs), preferably in the range of from 0.15 to 0.6 bar(abs), more preferably in the range of from 0.2 to 0.4 bar(abs).

Embodiment 25: The process of any one of embodiments 1 to 24, wherein from 99 to 100 weight-%, more preferably from 99.5 to 100 weight-%, more preferably from 99.9 to 100 weight-% of the stream H(1) consist of water and A(1)OH, wherein preferably from 37.5 to 58 weight-%, more preferably from 40 to 55 weight-%, more preferably from 42.5 to 52 weight-% of the stream H(1) consist of A(1)OH.

Embodiment 26: The process of any one of embodiments 1 to 25, wherein the stream H(1) is fed into the reactive distillation column K(1) at a temperature of H(1) in the range of from ambient temperature to its boiling temperature, preferably in the range of from 50 to 80 °C.

Embodiment 27: The process of any one of embodiments 1 to 26, wherein the stream H(1) is fed into the top of the reactive distillation column K(1), preferably to the first theoretical stage from the top.

Embodiment 28: The process of any one of embodiments 1 to 27, wherein the reactive distillation column K(1) has from 5 to 50, preferably from 10 to 40, more preferably from 15 to 30 theoretical stages, wherein the stream G(1) is fed into the reactive distillation column K(1) at a position preferably between the bottoms and the fifth theoretical stage, more preferably between the bottoms and the third theoretical stage, more preferably between the bottoms and the second theoretical stage of the reactive distillation column K(1).

Embodiment 29: The process of any one of embodiments 1 to 28, wherein the reactive distillation column K(1) is operated at a pressure at the top in the range of from 0.5 to 10 bar(abs), preferably in the range of from 1 to 6 bar(abs), more preferably in the range of from 1 to 5 bar(abs).

Embodiment 30: The process of any one of embodiments 1 to 29, wherein the reactive distillation column K(1) is operated at a reflux ratio of 0:1.

Embodiment 31: The process of any one of embodiments 1 to 30, wherein from 99 to 100 weight-%, preferably from 99.5 to 100 weight-%, more preferably from 99.9 to 100 weight-% of the stream W(1) consist of methanol and water, wherein preferably from 1 to 10 weight-%, more preferably from 2 to 8 weight-%, more preferably from 5 to 6 weight-% of the stream W(1) consist of water.

Embodiment 32: The process of any one of embodiments 1 to 31, wherein from 99 to 100 weight-%, preferably from 99.5 to 100 weight-%, more preferably from 99.9 to 100 weight-% of the stream P(1) consist of A(1)OMe and methanol, wherein preferably from 10 to 50 weight-%, more preferably from 20 to 40 weight-%, more preferably from 25 to 35 weight-% of the stream P(1) consist of A(1)OMe, and wherein preferably at most 5000 weight-ppm, more preferably at most 2000 weight-ppm, more preferably at most 1000 weight-ppm of the stream P(1) consist of water.

Embodiment 33: The process of any one of embodiments 1 to 32, wherein the reactive distillation column K(1) is operated at a reboiler duty allowing to achieve a specific concentration of A(1)OMe in the stream P(1), preferably the concentration as defined in embodiment 39.

Embodiment 34: The process of any one of embodiments 1 to 33, wherein the top of the reactive distillation column K(1) is equipped with a droplet separating device D(1), preferably a demister, the process comprising separating droplets comprising A(1)OH from the vapor stream in the top of K(1).

Embodiment 35: The process of embodiment 34, comprising at least temporarily spraying the demister, preferably with a stream M(1) comprising methanol, said stream preferably being branched from a condensed top stream removed from the rectification column D or being a fresh methanol stream.

Embodiment 36: The process of any one of embodiments 1 to 35, wherein from 99 to 100 weight-%, more preferably from 99.5 to 100 weight-%, more preferably from 99.9 to 100 weight-% of the stream H(2) consist of water and A(2)OH, wherein preferably from 30 to 55 weight-%, more preferably from 40 to 52.5 weight-%, more preferably from 45 to 50 weight-% of the stream H(2) consist of A(2)OH .

Embodiment 37: The process of any one of embodiments 1 to 36, wherein the stream H(2) is fed into the reactive distillation column K(2) at a temperature of H(2) in the range of from ambient temperature to its boiling temperature, preferably in the range of from 50 to 80 °C

Embodiment 38: The process of any one of embodiments 1 to 37, wherein the stream H(2) is fed into the top of the reactive distillation column K(2), preferably to the first theoretical stage from the top.

Embodiment 39: The process of any one of embodiments 1 to 38, wherein the reactive distillation column K(2) has from 5 to 50, preferably from 10 to 40, more preferably from 15 to 30 theoretical stages, wherein the stream G(2) is fed into the reactive distillation column K(2) at a position preferably between the bottoms and the fifth theoretical stage, more preferably between the bottoms and the third theoretical stage, more preferably between the bottoms and the second theoretical stage of the reactive distillation column K(2).

Embodiment 40: The process of any one of embodiments 1 to 39, wherein the reactive distillation column K(2) is operated at a pressure at the top in the range of from 0.5 to 10 bar(abs), preferably in the range of from 1 to 6 bar(abs), more preferably in the range of from 1 to 5 bar(abs)

Embodiment 41: The process of any one of embodiments 1 to 40, wherein the reactive distillation column K(2) is operated at a reflux ratio of 0:1.

Embodiment 42: The process of any one of embodiments 1 to 41, wherein from 99 to 100 weight-%, preferably from 99.5 to 100 weight-%, more preferably from 99.9 to 100 weight-% of the stream W(2) consist of methanol and water, wherein preferably from 1 to 15 weight-%, more preferably from 2 to 12 weight-%, more preferably from 6 to 10 weight-% of the stream W(2) consist of water.

Embodiment 43: The process of any one of embodiments 1 to 42, wherein from 99 to 100 weight-%, preferably from 99.5 to 100 weight-%, more preferably from 99.9 to 100 weight-% of the stream P(2) consist of A(2)OMe and methanol, wherein preferably from 10 to 50 weight-%, more preferably from 20 to 40 weight-%, more preferably from 25 to 35 weight-% of the stream P(2) consist of A(2)OMe, and wherein preferably at most 5000 weight-ppm, more preferably at most 2000 weight-ppm, more preferably at most 1000 weight-ppm of the stream P(2) consist of water.

Embodiment 44: The process of any one of embodiments 1 to 43, wherein the reactive distillation column K(2) is operated at a reboiler duty allowing to achieve a specific concentration of A(2)OMe in the stream P(2), preferably the concentration as defined in embodiment 50.

Embodiment 45: The process of any one of embodiments 1 to 44, wherein the top of the reactive distillation column K(2) is equipped with a droplet separating device D(2), preferably a demister, the process comprising separating droplets comprising A(2)OH from the vapor stream in the top of K(2).

Embodiment 46: The process of embodiment 45, comprising at least temporarily spraying the demister, preferably with a stream M(2) comprising methanol, said stream preferably being branched from a condensed top stream removed from the rectification column D or being a fresh methanol stream.

Embodiment 47: The process of any one of embodiments 1 to 46, wherein prior to being fed into the rectification column D, the stream W(1) is passed through a compressor C(1), thereby realizing a pressure increase of W(1) in the range of from 0.1 to 0.8 bar(abs), preferably in the range of from 0.15 to 0.6 bar(abs), more preferably in the range of from 0.2 to 0.4 bar(abs).

Embodiment 48: The process of any one of embodiments 1 to 47, wherein prior to being fed into the rectification column D, the stream W(2) is passed through a compressor C(2), thereby realizing a pressure increase of W(2) in the range of from 0.1 to 0.8 bar(abs), preferably in the range of from 0.15 to 0.6 bar(abs), more preferably in the range of from 0.2 to 0.4 bar(abs).

Embodiment 49: The process of embodiment 47 or 48, wherein prior to dividing according to (b), the stream G is not passed through a compressor C, preferably not passed through a compressor C thereby realizing a pressure increase of G in the range of from 0.1 to 0.8 bar(abs), preferably in the range of from 0.15 to 0.6 bar(abs), more preferably in the range of from 0.2 to 0.4 bar(abs).

Embodiment 50: The process of any one of embodiments 47 to 49, wherein prior to be fed into the reactive distillation column K(1), the stream G(1) is not passed through a compressor C(1), preferably not passed through a compressor C(1) thereby realizing a pressure increase of G(1) in the range of from 0.1 to 0.8 bar(abs), preferably in the range of from 0.15 to 0.6 bar(abs), more preferably in the range of from 0.2 to 0.4 bar(abs).

Embodiment 51: The process of any one of embodiments 47 to 50, wherein prior to be fed into the reactive distillation column K(2), the stream G(2) is not passed through a compressor C(2), preferably not passed through a compressor C(2) thereby realizing a pressure increase of G(2) in the range of from 0.1 to 0.8 bar(abs), preferably in the range of from 0.15 to 0.6 bar(abs), more preferably in the range of from 0.2 to 0.4 bar(abs).

Embodiment 52: The process of any one of embodiments 1 to 51, being an integrated process for simultaneously preparing 2 mixtures P(i), wherein the mixture P(1) comprises sodium methoxide as A(1)OMe and methanol and the mixture P(2) comprising potassium methoxide as A(2)OMe and methanol.

Embodiment 53: The process of any one of embodiments 1 to 51, being an integrated process for simultaneously preparing three mixtures P(1), P(2) and P(3), wherein the mixture P(1) comprises sodium methoxide as A(1)OMe and methanol, the mixture P(2) comprises potassium methoxide as A(2)OMe and methanol, and the mixture P(3) comprises lithium methoxide as A(3)OMe and methanol, the process comprising

> (a) providing a stream G comprising methanol;
> (b) dividing the stream G into three streams G(1), G(2) and G(3), G(1), G(2) and G(3) having the same composition as G;
> (c.1) preparing sodium methoxide comprising
>
> > (c.1.1) feeding the stream G(1) into the lower part of a reactive distillation column K(1), and feeding a liquid stream H(1), preferably an aqueous liquid stream H(1), H(1) comprising dissolved sodium hydroxide, into the upper part of the reactive distillation column K(1);
> > (c.1.2) subjecting G(1) and H(1) in K(1) to reactive distillation conditions, obtaining a top stream W(1) comprising methanol and water; and obtaining a bottoms stream P(1) comprising sodium methoxide and methanol;
>
> (c.2) preparing potassium methoxide comprising
>
> > (c.2.1) feeding the stream G(2) into the lower part of a reactive distillation column K(2), K(2) being arranged in parallel with K(1), and feeding a liquid stream H(2), preferably an aqueous liquid stream H(2), H(2) comprising dissolved potassium hydroxide, into the upper part of the reactive distillation column K(2);
> > (c.2.2) subjecting G(2) and H(2) in K(2) to reactive distillation conditions, obtaining a top stream W(2) comprising methanol and water; and obtaining a bottoms stream P(2) comprising potassium methoxide and methanol;
>
> (c.3) preparing lithium methoxide comprising
>
> > (c.3.1) feeding the stream G(3) into the lower part of a reactive distillation column K(3), K(3) being arranged in parallel with K(1) and K(2), and feeding a liquid stream H(3), preferably an aqueous liquid stream H(3), H(3) comprising dissolved lithium hydroxide, into the upper part of the reactive distillation column K(3);
> > (c.3.2) subjecting G(3) and H(3) in (K3) to reactive distillation conditions, obtaining a top stream W(3) comprising methanol and water; and obtaining a bottoms stream P(3) comprising lithium methoxide and methanol;
>
> (d.1) feeding W(1), W(2) and W(3) into the lower part of a rectification column D, and feeding a stream M comprising methanol into the upper part of the rectification column D;
> (d.2) subjecting W(1), W(2), W(3) and M in D to distillation conditions, obtaining the stream G according to (a) as a top stream.

Embodiment 54: The process of any one of embodiments 1 to 43, wherein from at least one of the streams P(i), A(i)OMe is at least partially separated from methanol, preferably obtaining solid, more preferably crystalline A(i)OMe.

Embodiment 55: A chemical production unit for carrying out the process according to any one of embodiments 1 to 54, comprising

- a rectification column D comprising

  -- inlet means for feeding the stream M into D;
  -- in its lower part, inlet means for feeding the streams W(i) or one or more combined stream thereof into D;
  -- outlet means for removing the streams T(2) and G or a combined stream thereof from the top of D;
  -- at least one condenser, preferably a condenser V(4) and optionally a further condenser V(5) arranged downstream of V(4), having inlet means for receiving the stream T(2) and having outlet means for removing the condensed stream T(3) and for removing a waste gas stream;
  -- inlet means for feeding the stream T(3) to the top of D;
  -- a bottom reboiler;

- a stream dividing device S for dividing the stream G into n streams G(i);
- means for passing the stream G to said stream dividing device S;
- n reactive distillation columns K(i), n≥2 and i=1...n; said reactive distillation columns K(i) being arranged in parallel, each reactive distillation column K(i) comprising

  -- in its upper part, preferably in its top, inlet means for feeding a stream H(i) into K(i);
  -- in its lower part, inlet means for feeding a stream G(i) into K(i);
  -- outlet means for removing a stream W(i) from the top of K(i);
  -- a bottom reboiler ;
  -- outlet means for removing a bottoms stream from K(i);
  -- a stream dividing means for separating a stream P(i) from the bottoms stream removed from K(i);

- means for passing the streams G(i) to the reactive distillation columns K(i);
- means for passing the streams W(i) to the rectification column D;
- one or more compressors C(i) for compressing either the stream G and/or the streams G(i) and/or the streams W(i);

the unit further comprising means for realizing recompression of the top vapor obtained from D, said means comprising a compressor C(3) for compressing a stream T(1) removed from the top of D, means for passing T(1) from the top of D to C(3), a reboiler V(6) for condensing said compressed stream, means for passing said compressed stream from (C3) to V(6), and means for feeding the obtained liquid stream to the top of D, wherein the reboiler V(6) is an intermediate reboiler of D.

Embodiment 56: The unit of embodiment 55, wherein the top of at least one, preferably of each reactive distillation column K(i) is equipped with a droplet separating device D(i), preferably a demister, said demister preferably comprising an inlet means for feeding a stream M(i) comprising methanol into said demister.

Embodiment 57: The unit of embodiment 55 or56, wherein each of the reactive distillations columns K(i) comprises, independently from one another, from 5 to 50, preferably from 10 to 40, more preferably from 15 to 30 theoretical stages.

Embodiment 58: The unit of any one of embodiments 55 to 57, wherein the means for passing the streams G(i) to the reactive distillation columns K(i) are located, independently from one another, at a position between the bottoms and the fifth theoretical stage, more preferably between the bottoms and the third theoretical stage, more preferably between the bottoms and the second theoretical stage of K(i).

Embodiment 59: The unit of any one of embodiments 55 to 58, wherein the means for passing the streams H(i) into the reactive distillation columns K(i) are located at the top of K(i), preferably at the uppermost theoretical stage.

Embodiment 60: The unit of any one of embodiments 55 to 59, wherein at least one, preferably each reactive distillation columns K(i) does not comprise means for being operated at a reflux ratio of greater than 0:1.

Embodiment 61: The unit of any one of embodiments 55 to 60, wherein each reactive distillation column K(i) is equipped with trays.

Embodiment 62: The unit of any one of embodiments 55 to 61, comprising n compressors C(i) arranged upstream of K(i) for compressing the streams G(i).

Embodiment 63: The unit of any one of embodiments 55 to 61, comprising n compressors C(i) arranged downstream of K(i) and upstream of D for compressing the streams W(i).

Embodiment 64: The unit of any one of embodiments 55 to 63, wherein the rectification column D has from 20 to 100, preferably from 30 to 80, more preferably from 40 to 60 theoretical stages.

Embodiment 65: The unit of any one of embodiments 55 to 64, wherein the inlet means for feeding the streams W(i) or one or more combined stream thereof into D is/are located at a position between the bottoms and the 15th theoretical stage, preferably between the bottoms and the 10th theoretical stage, more preferably between the bottoms and the 8th theoretical of D.

Embodiment 66: The unit of any one of embodiments 55 to 65, wherein the inlet means for feeding the stream M into D are located in the upper part of D, preferably at least 4 theoretical stages from the top of D, more preferably between the 4th and the 20th theoretical stage from the top of D, more preferably between the 6th and the 15th theoretical stage from the top of D.

Embodiment 67: The unit of any one of embodiments 55 to 66, further comprising at least one condensate drum for a liquid stream removed from V(4) and optionally from V(5) and further comprising means for passing at least part of the liquid contained in said drum as the stream T(3) to the top of D.

Embodiment 68: The unit of any one of embodiments 55 to 67, wherein the rectification column is equipped with trays and/or packings, wherein, insofar as embodiment 77 is dependent on embodiment 76, D is equipped with packings arranged above the intermediate reboiler of D and with trays arranged below the intermediate reboiler of D.

Embodiment 69: The unit of any one of embodiments 55 to 68, further comprising at least one condensate drum for the condensed stream removed from V(6), the unit preferably further comprising means for passing at least part of a gas phase in said drum to V(4) and means for passing at least part of a liquid phase in said drum to a condensate drum according to embodiment 74.

Embodiment 70: The unit of any one of embodiments 55 to 69, further comprising means for separating an alkali metal methoxide A(i)OMe from at least one of the streams P(i).

Embodiment 71: The unit of any one of embodiments 55 to 70, wherein n is in the range of from 2 to 10, preferably in the range of from 2 to 5, more preferably 2 or 3, more preferably 2.

Embodiment 72: Use of a chemical production unit according to any one of embodiments 62 to 80 or of a process according to any one of embodiments 1 to 54 for simultaneously producing n mixtures P(i) comprising alkali metal methoxide and methanol, n being an integer with n≥2 and i=1...n, wherein

either at least 2 of the mixtures P(i) comprise different alkali metal methoxides A(i)OMe,

and/or at least 2 of the mixtures P(i) comprise the same alkali metal alkoxide A(i)OMe at different concentrations.

The present invention is further illustrated by the following examples 1 and 2 and the figures 1 to 6.

Examples

Comparative Example 1: Simultaneous production of sodium methoxide and potassium methoxide without top vapor recompression in rectification column D

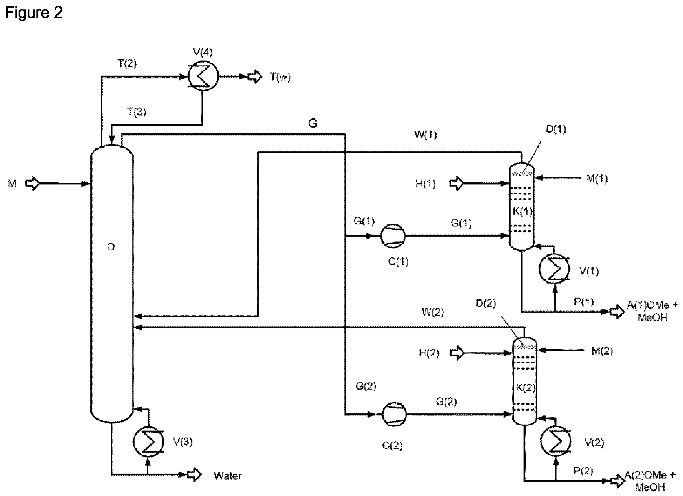

Fig. 2 shows a process scheme for preparing a mixture P(1) comprising NaOMe and MeOH and a mixture P(2) comprising KOMe and MeOH. Regarding the operating conditions of the rectification column D and of the reactive distillation columns K(1) and K(2), reference is made to Table 1a below. Regarding the relative mass flow rates, reference is made to Table 1b below.

Table 1a

| Operating conditions of the columns D, K(1) and K(2) | | |
|---|---|---|
| Column D | Pressure at the top / bar(abs) | 2.1 |
| | Temperature at the top / °C | 84 |
| | Pressure at the bottom / bar(abs) | 2.23 |
| | Temperature at the bottom / °C | 124 |
| | Theoretical stages | 50 |
| | W(1), W(2) to theoretical stage from bottom | 8th |
| | M fed to theoretical stage from bottom | 42th |
| | | |

(continued)

| Operating conditions of the columns D, K(1) and K(2) | | |
|---|---|---|
| Column K(1) | Pressure at the top / bar(abs) | 2.15 |
| | Temperature at the top / °C | 89 |
| | Pressure at the bottom / bar(abs) | 2.3 |
| | Temperature at the bottom / °C | 117 |
| | Number of trays | 40 |
| | | |
| Column K(2) | Pressure at the top / bar(abs) | 2.15 |
| | Temperature at the top / °C | 89 |
| | Pressure at the bottom / bar(abs) | 2.3 |
| | Temperature at the bottom / °C | 116 |
| | Number of trays | 40 |

Table 1b

| Relationships between the mass flow rates f of the different streams | | |
|---|---|---|
| Definition of streams | Specified: H(1), H(2) | |
| | M(1), M(2): | part of condensate from V(4) |
| | H(1): | NaOH 50 weight-% in water |
| | H(2): | KOH 48 weight-% in water |
| | | |
| Ratios of mass flow rates of streams | f(G(1)) / f(H(1)) | 13.3 |
| | f(G(2)) / f(H(2)) | 9.03 |
| | f(M(1)) / f(H(1)) | 0.55 |
| | f(M(2)) / f(H(2)) | 0.40 |
| | f(T(3)) / f(G) *) | 0.92 |
| | f(P(1)) / f(H(1)) | 2.25 |
| | f(P(2)) / f(H(2)) | 1.86 |
| | f(waste gas) / f(G) *) | < 0.0015 |
| *) $$f(G) = f(G(1)) + f(G(2))$$ P(1): 30 weight-% of sodium methoxide in methanol, < 1000 ppm of water. P(2): 32 weight-% of potassium methoxide in methanol, < 1000 ppm of water. | | |

In the following, it is indicated how the mass flow rate of the methanol contained in the stream M (methanol balance, fresh methanol stream), $f_{MeOH}(M)$, is calculated. In this calculation, the water contents of P(1) and P(2), both being less than 1000 weight-ppm, are neglected. According to this calculation, $f_{MeOH}(P(1))$ is the mass flow rate of MeOH contained in the stream P(1), $f_{MeOH}(P(2))$ is the mass flow rate of MeOH contained in the stream P(2), $f_{MeOH}(water)$ is the mass flow rate of MeOH contained in the water stream, and $f_{MeOH}(waste gas)$ is the mass flow rate of MeOH contained in the waste gas stream:

$$f_{MeOH}(M) = f_{MeOH}(P(1)) + f_{MeOH}(P(2)) + f_{MeOH}(water) + f_{MeOH}(waste gas)$$

1.1

$$f_{MeOH}(P(1)) = [(1-c_{NaOME}) * f(P(1))] + [(M_{MeOH}/M_{NaOH} * c_{NaOH}) * f(H(1))]$$

| Molecular mass M / concentration c | values | units |
|---|---|---|
| | | |
| $M_{MeOH}$ | 32 | kg/kmol |
| $M_{NaOH}$ | 40 | kg/kmol |
| $C_{NaOH}$ in H(1) | 0.5 | kg/kg |
| $C_{NaOMe}$ in P(1) | 0.3 | kg/kg |

1.2

$$f_{MeOH}(P(2)) = [(1-c_{KOMe}) * f(P(2))] + [(M_{MeOH}/M_{KOH} * c_{KOH}) * f(H(2))]$$

| Molecular mass M / concentration c | values | units |
|---|---|---|
| | | |
| $M_{MeOH}$ | 32 | kg/kmol |
| $M_{KOH}$ | 56 | kg/kmol |
| $C_{KOH}$ in H(2) | 0.48 | kg/kg |
| $C_{KOMe}$ in P(2) | 0.32 | kg/kg |

1.3

$$f_{MeOH}(water) = 0.001 * f(water) \text{ (maximum value)}$$

1.4

$$f_{MeOH}(waste\ gas) = 0 \text{ (neglected)}$$

In the following, it is indicated how the mass flow rate of the water contained in the stream water (bottom stream of D, waste water stream), f(water), is calculated. In this calculation, the water contents of P(1) and P(2), both being less than 1000 weight-ppm, are neglected.

1.5

$$f(water) = f_{H2O}(H(1)) + f_{H2O}(H(2)) + f_{H2O}(M) - f_{H2O}(waste\ gas)$$

$f_{H2O}(H(1))$ is the mass flow rate of water contained in the stream H(1) and $f_{H2O}(H(2))$ is the mass flow rate of water contained in the stream H(2) and $f_{H2O}(M)$ is the mass flow rate of water contained in the stream M:

1.5.1

$$f_{H2O}(H(1)) = [(1-c_{NaOH}) * f(H(1))] + [(M_{H2O}/M_{NaOH} * c_{NaOH}) * f(H(1))]$$

| Molecular mass M / concentration c | values | units |
|---|---|---|
| | | |
| $M_{H2O}$ | 18 | kg/kmol |
| $M_{NaOH}$ | 40 | kg/kmol |
| $C_{NaOH}$ in H(1) | 0.5 | kg/kg |

1.5.2

$$f_{H2O}(H(2)) = [(1-c_{KOH}) * f(H(2))] + [(M_{H2O}/M_{KOH} * c_{KOH}) * f(H(2))]$$

| Molecular mass M / concentration c | values | units |
|---|---|---|
| | | |
| $M_{H2O}$ | 18 | kg/kmol |
| $M_{KOH}$ | 56 | kg/kmol |
| $C_{KOH}$ in H(2) | 0.48 | kg/kg |

1.5.3

$$f_{H2O}(M) = 0.001 * f(M)$$

1.5.4

$$f_{H2O}(\text{waste gas}) = 0 \text{ (neglected)}$$

**Example 1:** Simultaneous production of sodium methoxide and potassium methoxide with top vapor recompression in rectification column D

The use of vapor recompression reduces the energy demand of the distillation in D considerably. It is possible to have a ratio of the heat streams to V(3) and V(6) of about 1:4. That means, the energy demand decreases to 20 %. But about 10 % (depending on the pressure) of the energy which is transferred in V(6) is needed as power for the compressor C(3). All in all, there is a large energy saving by using vapor recompression.

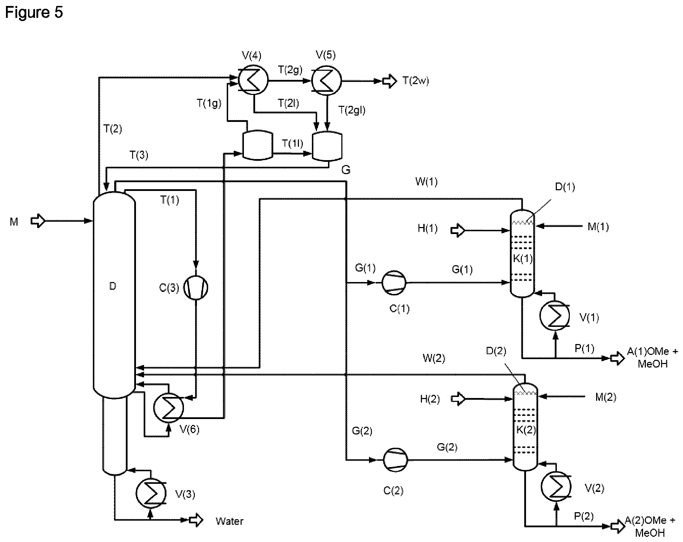

Fig. 5 shows a process scheme for preparing a mixture P(1) comprising NaOMe and MeOH and a mixture P(2) comprising KOMe and MeOH. Regarding the operating conditions of the rectification column D and of the reactive distillation columns K(1) and K(2), reference is made to Table 2a below. Regarding the relative mass flow rates, reference is made to Table 2b below.

**Table 2a**

| Operating conditions of the columns D, K(1) and K(2) | | |
|---|---|---|
| Column D | Pressure at the top / bar(abs) | 2.1 |
| | Temperature at the top / °C | 84 |
| | Pressure at the bottom / bar(abs) | 2.23 |
| | Temperature at the bottom / °C | 124 |
| | Theoretical stages | 50 |
| | W(1), W(2) to theoretical stage from bottom | 8th |
| | M fed to theoretical stage from bottom | 42th |
| | Pressure at outlet of C(3) / bar(abs) | 5 |
| | | |
| Column K(1) | Pressure at the top / bar(abs) | 2.15 |
| | Temperature at the top / °C | 89 |
| | Pressure at the bottom / bar(abs) | 2.3 |
| | Temperature at the bottom / °C | 117 |
| | Number of trays | 40 |
| | | |

(continued)

| Operating conditions of the columns D, K(1) and K(2) | | |
|---|---|---|
| Column K(2) | Pressure at the top / bar(abs) | 2.15 |
| | Temperature at the top / °C | 89 |
| | Pressure at the bottom / bar(abs) | 2.3 |
| | Temperature at the bottom / °C | 116 |
| | Number of trays | 40 |

**Table 2b**

| Relationships between the mass flow rates f of the different streams | | |
|---|---|---|
| Definition of streams | Specified: | H(1), H(2) |
| | M(1), M(2): | part of condensate from V(4) |
| | H(1): | NaOH 50 weight-% in water |
| | H(2): | KOH 48 weight-% in water |
| | | |
| Ratios of mass flow rates of streams | f(G(1)) / f(H(1)) | 13.3 |
| | f(G(2)) / f(H(2)) | 9.03 |
| | f(M(1)) / f(H(1)) | 0.55 |
| | f(M(2)) / f(H(2)) | 0.40 |
| | f(T(3)) / f(G) *) | 1.0 |
| | f(P(1)) / f(H(1)) | 2.25 |
| | f(P(2)) / f(H(2)) | 1.86 |
| | f(waste gas) / f(G) *) | < 0.0015 |
| | f(T(1)) / f(T(2)) | 3.97 |
| *) $$f(G) = f(G(1)) + f(G(2))$$ P(1): 30 weight-% of sodium methoxide in methanol, < 1000 ppm of water. P(2): 32 weight-% of potassium methoxide in methanol, < 1000 ppm of water. | | |

In the following, it is indicated how the mass flow rate of the methanol contained in the stream M (methanol balance, fresh methanol stream), $f_{MeOH}(M)$, is calculated. In this calculation, the water contents of P(1) and P(2), both being less than 1000 weight-ppm, are neglected. According to this calculation, $f_{MeOH}(P(1))$ is the mass flow rate of MeOH contained in the stream P(1), $f_{MeOH}(P(2))$ is the mass flow rate of MeOH contained in the stream P(2), $f_{MeOH}(water)$ is the mass flow rate of MeOH contained in the water stream, and $f_{MeOH}(waste\ gas)$ is the mass flow rate of MeOH contained in the waste gas stream:

$$f_{MeOH}(M) = f_{MeOH}(P(1)) + f_{MeOH}(P(2)) + f_{MeOH}(water) + f_{MeOH}(waste\ gas)$$

2.1

$$f_{MeOH}(P(1)) = [(1-c_{NaOME}) * f(P(1))] + [(M_{MeOH}/M_{NaOH} * c_{NaOH}) * f(H(1))]$$

| Molecular mass M / concentration c | values | units |
|---|---|---|
| | | |
| $M_{MeOH}$ | 32 | kg/kmol |

(continued)

| Molecular mass M / concentration c | values | units |
|---|---|---|
| $M_{NaOH}$ | 40 | kg/kmol |
| $C_{NaOH}$ in H(1) | 0.5 | kg/kg |
| $C_{NaOMe}$ in P(1) | 0.3 | kg/kg |

2.2

$$f_{MeOH}(P(2)) = [(1-c_{KOMe}) * f(P(2))] + [(M_{MeOH}/M_{KOH} * c_{KOH}) * f(H(2))]$$

| Molecular mass M / concentration c | values | units |
|---|---|---|
| | | |
| $M_{MeOH}$ | 32 | kg/kmol |
| $M_{KOH}$ | 56 | kg/kmol |
| $C_{KOH}$ in H(2) | 0.48 | kg/kg |
| $C_{KOMe}$ in P(2) | 0.32 | kg/kg |

2.3

$$f_{MeOH}(water) = 0.001 * f(water) \text{ (maximum value)}$$

2.4

$$f_{MeOH}(waste\ gas) = 0 \text{ (neglected)}$$

In the following, it is indicated how the mass flow rate of the water contained in the stream water (bottom stream of D, waste water stream), f(water), is calculated. In this calculation, the water contents of P(1) and P(2), both being less than 1000 weight-ppm, are neglected.

2.5

$$f(water) = f_{H2O}(H(1)) + f_{H2O}(H(2)) + f_{H2O}(M) - f_{H2O}(waste\ gas)$$

$f_{H2O}(H(1))$ is the mass flow rate of water contained in the stream H(1) and $f_{H2O}(H(2))$ is the mass flow rate of water contained in the stream H(2) and $f_{H2O}(M)$ is the mass flow rate of water contained in the stream M:

2.5.1

$$f_{H2O}(H(1)) = [(1-c_{NaOH}) * f(H(1))] + [(M_{H2O}/M_{NaOH} * c_{NaOH}) * f(H(1))]$$

| Molecular mass M / concentration c | values | units |
|---|---|---|
| | | |
| $M_{H2O}$ | 18 | kg/kmol |
| $M_{NaOH}$ | 40 | kg/kmol |
| $C_{NaOH}$ in H(1) | 0.5 | kg/kg |

2.5.2

$$f_{H_2O}(H(2)) = [(1-c_{KOH}) * f(H(2))] + [(M_{H_2O}/M_{KOH} * c_{KOH}) * f(H(2))]$$

| Molecular mass M / concentration c | values | units |
|---|---|---|
| | | |
| $M_{H_2O}$ | 18 | kg/kmol |
| $M_{KOH}$ | 56 | kg/kmol |
| $c_{KOH}$ in H(2) | 0.48 | kg/kg |

2.5.3

$$f_{H_2O}(M) = 0.001 * f(M)$$

2.5.4

$$f_{H_2O}(\text{waste gas}) = 0 \text{ (neglected)}$$

**Description of the figures**

**Figure 1** shows a schematic overview of a process not according to the present invention wherein the rectification column D is operated without top vapor recompression. In particular, a fresh methanol stream M is fed into the upper part of a rectification column D. From the top of the rectification column D, a gas stream T(2), a dry methanol stream, is removed which as passed through the condenser V(4), from which condenser V(4) a waste gas stream T(2w), essentially consisting of inerts and methanol, and a liquid stream T(3) are obtained. The liquid stream T(3) is fed back to the top of the column D. A part of the bottoms stream removed from the column D is fed into the bottom reboiler V(3) of D, the other part of the bottoms stream, essentially consisting of water, is disposed. Further, from the top of the column D, a dry methanol gas stream G is removed, in addition to T(2). This gas stream G, exhibiting a flow rate f(G), is divided into two streams G(1) and G(2), both having the same composition as G. The stream G(1) exhibits a flow rate f(G(1)), the stream G(2) exhibits a flow rate f(G(2)), wherein f(G(1))+f((G2))=f(G). The stream G(1) is then passed through a compressor C(1), and the thus compressed stream G(1) is then fed into the lower part of reactive distillation column K(1), wherein into the upper part of K(1), a liquid aqueous stream H(1) comprising a dissolved alkali metal hydroxide A(1)OH is fed. A part of the bottoms stream removed from the column K(1) is fed into the reboiler V(1) of K(1), the other part of the bottoms stream is the mixture P(1) comprising alkali metal methoxide A(1)OMe and methanol. From the top of the column K(1), which is operated without reflux, a gas stream W(1) essentially consisting of methanol and water is removed, wherein W(1) is fed into a lower part of the rectification column D. The stream G(2) is then passed through a compressor C(2), and the thus compressed stream G(2) is then fed into the lower part of reactive distillation column K(2), wherein into the upper part of K(2), a liquid aqueous stream H(2) comprising a dissolved alkali metal hydroxide A(2)OH is fed. A part of the bottoms stream removed from the column K(2) is fed into the reboiler V(2) of K(2), the other part of the bottoms stream is the mixture P(2) comprising alkali metal methoxide A(2)OMe and methanol. From the top of the column K(2), which is operated without reflux, a gas stream W(2) essentially consisting of methanol and water is removed, wherein W(2) is fed, together with W(1), into a lower part of the rectification column D.

**Figure 2** shows the schematic overview according to Figure 1, wherein in the top of the reactive distillation columns K(1) and K(2), droplet separating devices D(1) and D(2), preferably demisters, are located, with the streams M(1) and M(2) used for an at least temporary spraying.

**Figure 3** shows the schematic overview according to Figure 2, wherein the compressors C(1) and C(2) are not located upstream of K(1) and K(2) for compressing G(1) and G(2), but located downstream of K(1) and K(2) for compressing W(1) and W(2) prior to feeding W(1) and W(2) into D.

**Figure 4** shows a schematic overview of a process according to the present invention wherein, compared to the process shown in Figure 1, the rectification column D is according to the invention operated with top vapor recompression. Regarding said top vapor recompression, a dry methanol top stream is removed from the rectification column D, in addition to G and T(2). This gas stream T(1) is passed through compressor C(3), and this compressed stream T(1) is then passed through the intermediate reboiler V(6) of the column D. The thus obtained compressed and condensed stream T(1) obtained from V(6) is then passed to a first condensate drum. Inerts which are comprised in T(1) are removed as gas stream T(1g) from the first condensate drum via a control valve (not shown) and fed into the condenser V(4) into which also the top stream T(2) is fed. The other part of T(1), T(1l), is depressurized into a second condensate drum. Into said second condensate drum, a further stream T(2gl) is fed which is obtained from the condenser V(5) into which the stream T(2g) obtained from V(4), T(2l) is fed. Further, the stream T(2l) obtained from V(4) is fed in to the second condensate drum. From the second condensate drum, a liquid stream T(3) is removed and fed to the top of the column D.

**Figure 5** shows the schematic overview according to Figure 4, wherein in the top of the reactive distillation columns K(1) and K(2), droplet separating devices D(1) and D(2), preferably demisters, are located, with the streams M(1) and M(2) used for an at least temporary spraying.

**Figure 6** shows the schematic overview according to Figure 5, wherein the compressors C(1) and C(2) are not located upstream of K(1) and K(2) for compressing G(1) and G(2), but located downstream of K(1) and K(2) for compressing W(1) and W(2) prior to feeding W(1) and W(2) into D.

**Cited literature**

- US 2002/0183566 A1
- US 2008/0296786 A1
- WO 2013/168113 A1

**Claims**

1. An integrated process for simultaneously preparing n mixtures P(i) comprising alkali metal methoxide and methanol, comprising

   providing n reactive distillation columns K(i);
   providing n aqueous liquid streams H(i), a given stream H(i) comprising a dissolved alkali metal hydroxide A(i)OH, wherein n is an integer with $n \geq 2$ and i=1...n; and
   providing a rectification column D;
   the process further comprising

      (a) providing a stream G comprising methanol;
      (b) dividing the stream G into n streams G(i), each stream G(i) having the same composition as G;
      (c) preparing the one or more alkali metal methoxides comprising

         feeding each stream G(i) into the lower part of a respective reactive distillation column K(i), and feeding the aqueous liquid stream H(i) comprising the dissolved alkali metal hydroxide A(i)OH into the upper part of said reactive distillation column K(i);
         and
         subjecting G(i) and H(i) in each K(i) to reactive distillation conditions, obtaining n top streams W(i) comprising methanol and water; and obtaining n bottoms streams P(i) comprising alkali metal methoxide A(i)OMe and methanol;

      (d) feeding each stream W(i) into the lower part of the rectification column D, and feeding a stream M comprising methanol into the rectification column D; and subjecting the n streams W(i) and M in D to distillation conditions, obtaining the stream G according to (a) as a top stream;

      wherein the rectification column D is operated at a reflux ratio of at least 0.5:1, wherein the rectification column D is operated with top vapor recompression, wherein for realizing the reflux ratio, the process comprises

(i) removing, in addition to G, a top stream T(2) from the rectification column D, passing said stream T(2) through a condenser V(4), obtaining a liquid stream and a waste gas stream T(2w);

(ii) removing, in addition to G and T(2), a further top stream T(1) from the rectification column D, passing said stream T(2) through a compressor C(3), passing the thus compressed stream through a reboiler V(6), obtaining a liquid stream, wherein the reboiler V(6) is an intermediate reboiler of the rectification column D;

(iii) feeding the liquid streams obtained according to (i) and (ii) into the top of the rectification column D;

or

(i) removing, in addition to G, a top stream T(2) from the rectification column D, passing said stream T(2) through a condenser V(4), obtaining a liquid stream T(2l) and a gas stream T(2g); passing the gas stream T(2g) through a condenser V(5), obtaining a liquid stream T(2gl) and a waste gas stream T(2w); and combining the liquid streams T(2l) and (T2gl), obtaining a combined liquid stream T(2cl);

(ii) removing, in addition to G and T(2), a further top stream T(1) from the rectification column D, passing said stream T(2) through a compressor C(3), passing the thus compressed stream through a reboiler V(6), obtaining a liquid stream, wherein the reboiler V(6) is an intermediate reboiler of the rectification column D;

(iii) feeding the combined liquid stream obtained according to (i) and the liquid stream obtained according to (ii) into the top of the rectification column D.

2. The process of claim 1, wherein n is in the range of from 2 to 10, preferably in the range of from 2 to 5, more preferably 2 or 3, more preferably 2.

3. The process of claim 1 or 2, wherein each alkali metal hydroxide A(i)OH is preferably selected from the group consisting of lithium hydroxide, sodium hydroxide and potassium hydroxide, more preferably from the group consisting of sodium hydroxide and potassium hydroxide and wherein a given aqueous liquid stream H(i) comprises an alkali metal hydroxide A(i)OH dissolved in water, in methanol, or in a mixture comprising water and methanol, preferably in water.

4. The process of any one of claims 1 to 3, being an integrated process for simultaneously preparing at least 2 mixtures P(i), preferably 2 mixtures P(i), wherein the mixture P(1) comprises A(1)OMe, preferably sodium methoxide, and methanol and the mixture P(2) comprises A(2)OMe, preferably potassium methoxide, and methanol, the process comprising

(a) providing a stream G comprising methanol;

(b) dividing the stream G into at least two streams G(1) and G(2), G(1) and G(2) having the same composition as G;

(c.1) preparing A(1)OMe, preferably sodium methoxide, comprising

(c.1.1) feeding the stream G(1) into the lower part of a reactive distillation column K(1), and feeding an aqueous liquid stream H(1) comprising dissolved A(1)OH, preferably sodium hydroxide, into the upper part of the reactive distillation column K(1);

(c.1.2) subjecting G(1) and H(1) in K(1) to reactive distillation conditions, obtaining a top stream W(1) comprising methanol and water; and obtaining a bottoms stream P(1) comprising A(1)OMe, preferably sodium methoxide, and methanol;

(c.2) preparing A(2)OMe, preferably potassium methoxide, comprising

(c.2.1) feeding the stream G(2) into the lower part of a reactive distillation column K(2), K(2) being arranged in parallel with K(1), and feeding an aqueous liquid stream H(2) comprising dissolved A(2)OH, preferably potassium hydroxide, into the upper part of the reactive distillation column K(2);

(c.2.2) subjecting G(2) and H(2) in K(2) to reactive distillation conditions, obtaining a top stream W(2) comprising methanol and water; and obtaining a bottoms stream P(2) comprising A(2)OMe, preferably potassium methoxide, and methanol;

(d.1) feeding W(1) and W(2) into the lower part of a rectification column D, and feeding a stream M comprising methanol into the rectification column D;

(d.2) subjecting W(1), W(2) and M in D to distillation conditions, obtaining the stream G according to (a) as a top stream.

5. The process of claim 4, wherein W(1) and W(2) are fed as gas streams into the rectification column D, preferably at a position between the bottoms and the 15th theoretical stage, preferably between the bottoms and the 10th theoretical stage, more preferably between the bottoms and the 8th theoretical of the rectification column D.

6. The process of any one of claims 1 to 5, wherein from 99 to 100 weight-%, preferably from 99.5 to 100 weight-%, more preferably from 99.9 to 100 weight-% of the stream M consist of methanol and optionally water, wherein the amount of water comprised in the stream M is at most 2000 weight-ppm, more preferably at most 1500 weight-ppm, more preferably at most 1000 weight-ppm, wherein the stream M is preferably fed to the upper part of D, more preferably at least 4 theoretical stages from the top of D, more preferably between the 4th and the 20th theoretical stage from the top of D, more preferably between the 6th and the 15th theoretical stage from the top of D, preferably at a temperature of M in the range of from ambient temperature to the boiling point of methanol at the column pressure of D, more preferably at ambient temperature.

7. The process of any one of claims 1 to 6, wherein the rectification column D is operated at a reflux ratio in the range of from 0.55:1 to 1.4:1, preferably in the range of from 0.6:1 to 1.4:1.

8. The process of any one of claims 1 to 7, wherein the stream G provided according to (a) by obtaining from distillation according to (d.2) comprises methanol and water, wherein preferably from 99.95 to 100 weight-% of G consist of methanol and water, and wherein the water content of G is at most 200 weight-ppm, preferably at most 150 weight-ppm, more preferably at most 100 weight-ppm, wherein more preferably said water content is in the range of from 5 to 100 weight-ppm, more preferably in the range of from 10 to 100 weight-ppm, more preferably in the range of from 15 to 100 weight-ppm.

9. The process of any one of claims 1 to 8, wherein according to (b), the stream G is divided into the two streams G(1) and G(2).

10. The process of any one of claims 1 to 9, wherein prior to be fed into the reactive distillation column K(1), the stream G(1) is passed through a compressor C(1), thereby realizing a pressure increase of G(1) in the range of from 0.1 to 0.8 bar(abs), preferably in the range of from 0.15 to 0.6 bar(abs), more preferably in the range of from 0.2 to 0.4 bar(abs); and/or, preferably and
prior to be fed into the reactive distillation column K(2), the stream G(2) is passed through a compressor C(2), thereby realizing a pressure increase of G(2) in the range of from 0.1 to 0.8 bar(abs), preferably in the range of from 0.15 to 0.6 bar(abs), more preferably in the range of from 0.2 to 0.4 bar(abs);

11. The process of any one of claims 1 to 9, wherein prior to being fed into the rectification column D, the stream W(1) is passed through a compressor C(1), thereby realizing a pressure increase of W(1) in the range of from 0.1 to 0.8 bar(abs), preferably in the range of from 0.15 to 0.6 bar(abs), more preferably in the range of from 0.2 to 0.4 bar(abs); and/or, preferably and
prior to being fed into the rectification column D, the stream W(2) is passed through a compressor C(2), thereby realizing a pressure increase of W(2) in the range of from 0.1 to 0.8 bar(abs), preferably in the range of from 0.15 to 0.6 bar(abs), more preferably in the range of from 0.2 to 0.4 bar(abs).

12. A chemical production unit for carrying out the process according to any one of claims 1 to 11, comprising

   - a rectification column D comprising

      -- inlet means for feeding the stream M into D;
      -- in its lower part, inlet means for feeding the streams W(i) or one or more combined stream thereof into D;
      -- outlet means for removing the streams T(2) and G or a combined stream thereof from the top of D;
      -- at least one condenser, preferably a condenser V(4) and optionally a further condenser V(5) arranged downstream of V(4), having inlet means for receiving the stream T(2) and having outlet means for removing the condensed stream T(3) and for removing a waste gas stream;
      -- inlet means for feeding the stream T(3) to the top of D;
      -- a bottom reboiler;

   - a stream dividing device S for dividing the stream G into n streams G(i);
   - means for passing the stream G to said stream dividing device S;
   - n reactive distillation columns K(i), n≥2 and i=1...n; said reactive distillation columns K(i) being arranged in parallel, each reactive distillation column K(i) comprising

-- in its upper part, preferably in its top, inlet means for feeding a stream H(i) into K(i);
-- in its lower part, inlet means for feeding a stream G(i) into K(i);
-- outlet means for removing a stream W(i) from the top of K(i);
-- a bottom reboiler ;
-- outlet means for removing a bottoms stream from K(i);
-- a stream dividing means for separating a stream P(i) from the bottoms stream removed from K(i);

- means for passing the streams G(i) to the reactive distillation columns K(i);
- means for passing the streams W(i) to the rectification column D;
- one or more compressors C(i) for compressing either the stream G and/or the streams G(i) and/or the streams W(i);

the unit further comprising means for realizing recompression of the top vapor obtained from D, said means comprising a compressor C(3) for compressing a stream T(1) removed from the top of D, means for passing T(1) from the top of D to C(3), a reboiler V(6) for condensing said compressed stream, means for passing said compressed stream from (C3) to V(6), and means for feeding the obtained liquid stream to the top of D, wherein the reboiler V(6) is an intermediate reboiler of D.

13. The unit of claim 12, comprising n compressors C(i) arranged upstream of K(i) for compressing the streams G(i) or comprising n compressors C(i) arranged downstream of K(i) and upstream of D for compressing the streams W(i).

14. The unit of claim 12 or 13, wherein the inlet means for feeding the streams W(i) or one or more combined stream thereof into D is/are located at a position between the bottoms and the 15th theoretical stage, preferably between the bottoms and the 10th theoretical stage, more preferably between the bottoms and the 8th theoretical of D; and/or wherein the inlet means for feeding the stream M into D are located in the upper part of D, preferably at least 4 theoretical stages from the top of D, preferably between the 4th and the 20th theoretical stage from the top of D, more preferably between the 6th and the 15th theoretical stage from the top of D.

15. Use of a chemical production unit according to any one of claims 12 to 14 or of a process according to any one of claims 1 to 11 for simultaneously producing n mixtures P(i) comprising alkali metal methoxide A(i)OMe and methanol, n being an integer with n≥2 and i=1...n, wherein either at least 2 of the mixtures P(i) comprise different alkali metal methoxides A(i)OMe, and/or at least 2 of the mixtures P(i) comprise the same alkali metal alkoxide A(i)OMe at different concentrations.

**Patentansprüche**

1. Integriertes Verfahren zum gleichzeitigen Herstellen von n Gemischen P(i), die Alkalimethoxid und Methanol umfassen, Folgendes umfassend:

Bereitstellen von n Reaktivdestillationskolonnen K(i);
Bereitstellen von n wässrigen Flüssigkeitsströmen H(i), wobei ein gegebener Strom H(i) ein gelöstes Alkalihydroxid A(i)OH umfasst, wobei n eine ganze Zahl mit n ≥ 2 ist und i = 1...n; und
Bereitstellen einer Rektifikationskolonne **D;**
wobei das Verfahren ferner Folgendes umfasst:

(a) Bereitstellen eines Stroms G, der Methanol umfasst;
(b) Teilen des Stroms G in n Ströme G(i), wobei jeder Strom G(i) die gleiche Zusammensetzung wie G aufweist;
(c) Herstellen des einen oder der mehreren Alkalimethoxide, umfassend Einleiten jedes Stroms G(i) in den unteren Teil einer jeweiligen Reaktivdestillationskolonne K(i) und Einleiten des wässrigen Flüssigkeitsstroms H(i), der das gelöste Alkalihydroxid A(i)OH umfasst, in den oberen Teil der Reaktivdestillationskolonne K(i); und
Einwirkenlassen von Reaktivdestillationsbedingungen auf G(i) und H(i) in jeder K(i) und dadurch Erhalten von n Kopfströmen W(i), die Methanol und Wasser umfassen; und Erhalten von n Sumpfströmen P(i), die Alkalimethoxid A(i)OMe und Methanol umfassen;

(d) Einleiten jedes Stroms W(i) in den unteren Teil der Rektifikationskolonne D und Einleiten eines Stroms M, der Methanol umfasst, in die Rektifikationskolonne D; und Einwirkenlassen von Destillationsbedingungen auf die n Ströme W(i) und M in D und dadurch Erhalten des Stroms G gemäß (a) als Kopfstrom;

wobei die Rektifikationskolonne D mit einem Rücklaufverhältnis von wenigstens 0,5 : 1 betrieben wird, wobei die Rektifikationskolonne D mit Kopfdampfwiederverdichtung betrieben wird, wobei zum Realisieren des Rücklaufverhältnisses das Verfahren Folgendes umfasst:

(i) Entfernen - zusätzlich zu G - eines Kopfstroms T(2) aus der Rektifikationskolonne D, Leiten des Stroms T(2) durch einen Verflüssiger V(4) und dadurch Erhalten eines Flüssigkeitsstroms und eines Abgasstroms T(2w);
(ii) Entfernen - zusätzlich zu G und T(2) - eines weiteren Kopfstroms T(1) aus der Rektifikationskolonne D, Leiten des Stroms T(2) durch einen Verdichter C(3), Leiten des so verdichteten Stroms durch einen Verdampfer V(6) und dadurch Erhalten eines Flüssigkeitsstroms, wobei der Verdampfer V(6) ein Zwischenverdampfer der Rektifikationskolonne D ist;
(iii) Einleiten der gemäß (i) und (ii) erhaltenen Flüssigkeitsströme in den Kopf der Rektifikationskolonne D;

oder

(i) Entfernen - zusätzlich zu G - eines Kopfstroms T(2) aus der Rektifikationskolonne D, Leiten des Stroms T(2) durch einen Verflüssiger V(4) und dadurch Erhalten eines Flüssigkeitsstroms T(2l) und eines Gasstroms T(2g); Leiten des Gasstroms T(2g) durch einen Verflüssiger V(5) und dadurch Erhalten eines Flüssigkeitsstroms T(2gl) und eines Abgasstroms T(2w); und Kombinieren der Flüssigkeitsströme T(2l) und (T2gl) und dadurch Erhalten eines kombinierten Flüssigkeitsstroms T(2cl);
(ii) Entfernen - zusätzlich zu G und T(2) - eines weiteren Kopfstroms T(1) aus der Rektifikationskolonne D, Leiten des Stroms T(2) durch einen Verdichter C(3), Leiten des so verdichteten Stroms durch einen Verdampfer V(6) und dadurch Erhalten eines Flüssigkeitsstroms, wobei der Verdampfer V(6) ein Zwischenverdampfer der Rektifikationskolonne D ist;
(iii) Einleiten des gemäß (i) erhaltenen kombinierten Flüssigkeitsstroms und des gemäß (ii) erhaltenen Flüssigkeitsstroms in den Kopf der Rektifikationskolonne D.

2. Verfahren nach Anspruch 1, wobei n im Bereich von 2 bis 10, vorzugsweise im Bereich von 2 bis 5, liegt, bevorzugter 2 oder 3, bevorzugter 2, beträgt.

3. Verfahren nach Anspruch 1 oder 2, wobei jedes Alkalihydroxid A(i)OH vorzugsweise aus der Gruppe bestehend aus Lithiumhydroxid, Natriumhydroxid und Kaliumhydroxid, bevorzugter aus der Gruppe bestehend aus Natriumhydroxid und Kaliumhydroxid, ausgewählt ist und wobei ein gegebener wässriger Flüssigkeitsstrom H(i) ein Alkalihydroxid A(i)OH umfasst, das in Wasser, in Methanol oder in einem Gemisch, das Wasser und Methanol umfasst, vorzugsweise in Wasser, gelöst ist.

4. Verfahren nach einem der Ansprüche 1 bis 3, bei dem es sich um ein integriertes Verfahren zum gleichzeitigen Herstellen von wenigstens 2 Gemischen P(i), vorzugsweise 2 Gemischen P(i), handelt, wobei das Gemisch P(1) A(1)OMe, vorzugsweise Natriummethoxid, und Methanol umfasst und das Gemisch P(2) A(2)OMe, vorzugsweise Kaliummethoxid, und Methanol umfasst, wobei das Verfahren Folgendes umfasst:

(a) Bereitstellen eines Stroms G, der Methanol umfasst;
(b) Teilen des Stroms G in wenigstens zwei Ströme G(1) und G(2), wobei G(1) und G(2) die gleiche Zusammensetzung wie G aufweisen;
(c.1) Herstellen von A(1)OMe, vorzugsweise Natriummethoxid, umfassend:
(c.1.1) Einleiten des Stroms G(1) in den unteren Teil einer Reaktivdestillationskolonne K(1) und Einleiten eines wässrigen Flüssigkeitsstroms H(1), der gelöstes A(1)OH, vorzugsweise Natriumhydroxid, umfasst, in den oberen Teil der Reaktivdestillationskolonne K(1);
(c.1.2) Einwirkenlassen von Reaktivdestillationsbedingungen auf G(1) und H(1) in K(1) und dadurch Erhalten eines Kopfstroms W(1), der Methanol und Wasser umfasst; und Erhalten eines Sumpfstroms P(1), der A(1)OMe, vorzugsweise Natriummethoxid, und Methanol umfasst;
(c.2) Herstellen von A(2)OMe, vorzugsweise Kaliummethoxid, umfassend:
(c.2.1) Einleiten des Stroms G(2) in den unteren Teil einer Reaktivdestillationskolonne K(2), wobei K(2) parallel zu K(1) angeordnet ist, und Einleiten eines wässrigen Flüssigkeitsstroms H(2), der gelöstes A(2)OH, vorzugsweise Kaliumhydroxid, umfasst, in den oberen Teil der Reaktivdestillationskolonne K(2);

(c.2.2) Einwirkenlassen von Reaktivdestillationsbedingungen auf G(2) und H(2) in K(2) und dadurch Erhalten eines Kopfstroms W(2), der Methanol und Wasser umfasst; und Erhalten eines Sumpfstroms P(2), der A(2)OMe, vorzugsweise Kaliummethoxid, und Methanol umfasst;

(d.1) Einleiten von W(1) und W(2) in den unteren Teil einer Rektifikationskolonne D und Einleiten eines Stroms M, der Methanol umfasst, in die Rektifikationskolonne D;

(d.2) Einwirkenlassen von Destillationsbedingungen auf W(1), W(2) und M in D und dadurch Erhalten des Stroms G gemäß (a) als Kopfstrom.

5. Verfahren nach Anspruch 4, wobei W(1) und W(2) als Gasströme in die Rektifikationskolonne D eingeleitet werden, vorzugsweise an einer Position zwischen dem Sumpf und der 15. theoretischen Stufe, vorzugsweise zwischen dem Sumpf und der 10. theoretischen Stufe, bevorzugter zwischen dem Sumpf und der 8. theoretischen Stufe, der Rektifikationskolonne D.

6. Verfahren nach einem der Ansprüche 1 bis 5, wobei 99 bis 100 Gew.-%, vorzugsweise 99,5 bis 100 Gew.-%, bevorzugter 99,9 bis 100 Gew.-%, des Stroms M aus Methanol und optional Wasser bestehen, wobei die Menge an Wasser, die in dem Strom M enthalten ist, höchstens 2000 Gew.-ppm, bevorzugter höchstens 1500 Gew.-ppm, bevorzugter höchstens 1000 Gew.-ppm, beträgt, wobei der Strom M vorzugsweise in den oberen Teil von D eingeleitet wird, bevorzugter wenigstens 4 theoretische Stufen vom Kopf von D entfernt, bevorzugter zwischen der 4. und der 20. theoretischen Stufe ausgehend vom Kopf von D, bevorzugter zwischen der 6. und der 15. theoretischen Stufe ausgehend vom Kopf von D, vorzugsweise bei einer Temperatur von M im Bereich von Umgebungstemperatur bis zum Siedepunkt von Methanol beim Kolonnendruck von D, bevorzugter bei Umgebungstemperatur.

7. Verfahren nach einem der Ansprüche 1 bis 6, wobei die Rektifikationskolonne D mit einem Rücklaufverhältnis im Bereich von 0,55 : 1 bis 1,4 : 1, vorzugsweise im Bereich von 0,6 : 1 bis 1,4 : 1, betrieben wird.

8. Verfahren nach einem der Ansprüche 1 bis 7, wobei der Strom G, der gemäß (a) durch Erhalten aus Destillation gemäß (d.2) bereitgestellt wird, Methanol und Wasser umfasst, wobei vorzugsweise 99,95 bis 100 Gew.-% von G aus Methanol und Wasser bestehen und wobei der Wassergehalt von G höchstens 200 Gew.-ppm, vorzugsweise höchstens 150 Gew.-ppm, bevorzugter höchstens 100 Gew.-ppm, beträgt, wobei bevorzugter der Wassergehalt im Bereich von 5 bis 100 Gew.-ppm, bevorzugter im Bereich von 10 bis 100 Gew.-ppm, bevorzugter im Bereich von 15 bis 100 Gew.-ppm, liegt.

9. Verfahren nach einem der Ansprüche 1 bis 8, wobei gemäß (b) der Strom G in die zwei Ströme G(1) und G(2) geteilt wird.

10. Verfahren nach einem der Ansprüche 1 bis 9, wobei vor dem Einleiten in die Reaktivdestillationskolonne K(1) der Strom G(1) durch einen Verdichter C(1) geleitet wird, wodurch eine Druckerhöhung von G(1) im Bereich von 0,1 bis 0,8 bar(abs), vorzugsweise im Bereich von 0,15 bis 0,6 bar(abs), bevorzugter im Bereich von 0,2 bis 0,4 bar(abs), realisiert wird;
und/oder, vorzugsweise und,
vor dem Einleiten in die Reaktivdestillationskolonne K(2) der Strom G(2) durch einen Verdichter C(2) geleitet wird, wodurch eine Druckerhöhung von G(2) im Bereich von 0,1 bis 0,8 bar(abs), vorzugsweise im Bereich von 0,15 bis 0,6 bar(abs), bevorzugter im Bereich von 0,2 bis 0,4 bar(abs), realisiert wird.

11. Verfahren nach einem der Ansprüche 1 bis 9, wobei vor dem Einleiten in die Rektifikationskolonne D der Strom W(1) durch einen Verdichter C(1) geleitet wird, wodurch eine Druckerhöhung von W(1) im Bereich von 0,1 bis 0,8 bar(abs), vorzugsweise im Bereich von 0,15 bis 0,6 bar(abs), bevorzugter im Bereich von 0,2 bis 0,4 bar(abs), realisiert wird;
und/oder, vorzugsweise und,
vor dem Einleiten in die Rektifikationskolonne D der Strom W(2) durch einen Verdichter C(2) geleitet wird, wodurch eine Druckerhöhung von W(2) im Bereich von 0,1 bis 0,8 bar(abs), vorzugsweise im Bereich von 0,15 bis 0,6 bar(abs), bevorzugter im Bereich von 0,2 bis 0,4 bar(abs), realisiert wird.

12. Chemische Produktionseinheit zum Durchführen des Verfahrens nach einem der Ansprüche 1 bis 11, umfassend:

- eine Rektifikationskolonne D, umfassend:

-- Einlassmittel zum Einleiten des Stroms M in D;

-- in ihrem unteren Teil, Einlassmittel zum Einleiten der Ströme W(i) oder eines oder mehrerer kombinierter Ströme davon in D;

-- Auslassmittel zum Entfernen der Ströme T(2) und G oder eines kombinierten Stroms davon aus dem Kopf von D;

-- wenigstens einen Verflüssiger, vorzugsweise einen Verflüssiger V(4) und optional einen weiteren Verflüssiger V(5), der V(4) nachgeschaltet angeordnet ist, mit Einlassmitteln zum Empfangen des Stroms T(2) und mit Auslassmitteln zum Entfernen des verflüssigten Stroms T(3) und zum Entfernen eines Abgasstroms;

-- Einlassmittel zum Einleiten des Stroms T(3) in den Kopf von D;

-- einen Sumpfverdampfer;

- eine Stromteilungsvorrichtung S zum Teilen des Stroms G in n Ströme G(i);
- Mittel zum Leiten des Stroms G zu der Stromteilungsvorrichtung S;
- n Reaktivdestillationskolonnen K(i), n ≥ 2 und i = 1...n; wobei die Reaktivdestillationskolonnen K(i) parallel angeordnet sind, wobei jede Reaktivdestillationskolonne K(i) Folgendes umfasst:

-- in ihrem oberen Teil, vorzugsweise in ihrem Kopf, Einlassmittel zum Einleiten eines Stroms H(i) in K(i);

-- in ihrem unteren Teil, Einlassmittel zum Einleiten eines Stroms G(i) in K(i);

-- Auslassmittel zum Entfernen eines Stroms W(i) aus dem Kopf von K(i);

-- einen Sumpfverdampfer;

-- Auslassmittel zum Entfernen eines Sumpfstroms aus K(i);

-- ein Stromteilungsmittel zum Abtrennen eines Stroms P(i) von dem aus K(i) entfernten Sumpfstrom;

- Mittel zum Leiten der Ströme G(i) zu den Reaktivdestillationskolonnen K(i);
- Mittel zum Leiten der Ströme W(i) zu der Rektifikationskolonne D;
- einen oder mehrere Verdichter C(i) zum Verdichten entweder des Stroms G und/oder der Ströme G(i) und/oder der Ströme W(i);

wobei die Einheit ferner Mittel zum Realisieren einer Wiederverdichtung des aus D erhaltenen Kopfdampfes umfasst, wobei die Mittel einen Verdichter C(3) zum Verdichten eines aus dem Kopf von D entfernten Stroms T(1), Mittel zum Leiten von T(1) vom Kopf von D zu C(3), einen Verdampfer V(6) zum Verflüssigen des verdichteten Stroms, Mittel zum Leiten des verdichteten Stroms von (C3) zu V(6) und Mittel zum Einleiten des erhaltenen Flüssigkeitsstroms in den Kopf von D umfassen, wobei der Verdampfer V(6) ein Zwischenverdampfer von D ist.

13. Einheit nach Anspruch 12, umfassend n Verdichter C(i), die K(i) vorgeschaltet angeordnet sind, zum Verdichten der Ströme G(i) oder umfassend n Verdichter C(i), die K(i) nachgeschaltet und D vorgeschaltet angeordnet sind, zum Verdichten der Ströme W(i).

14. Einheit nach Anspruch 12 oder 13, wobei das/die Einlassmittel zum Einleiten der Ströme W(i) oder eines oder mehrerer kombinierter Ströme davon in D sich an einer Position zwischen dem Sumpf und der 15. theoretischen Stufe, vorzugsweise zwischen dem Sumpf und der 10. theoretischen Stufe, bevorzugter zwischen dem Sumpf und der 8. theoretischen Stufe, von D befindet/befinden; und/oder

wobei die Einlassmittel zum Einleiten des Stroms M in D sich im oberen Teil von D befinden, vorzugsweise wenigstens 4 theoretische Stufen vom Kopf von D entfernt, vorzugsweise zwischen der 4. und der 20. theoretischen Stufe ausgehend vom Kopf von D, bevorzugter zwischen der 6. und der 15. theoretischen Stufe ausgehend vom Kopf von D.

15. Verwendung einer chemischen Produktionseinheit nach einem der Ansprüche 12 bis 14 oder eines Verfahrens nach einem der Ansprüche 1 bis 11 zum gleichzeitigen Herstellen von n Gemischen P(i), die Alkalimethoxid A(i)OMe und Methanol umfassen, wobei n eine ganze Zahl mit n ≥ 2 ist und i = 1...n, wobei

entweder wenigstens 2 der Gemische P(i) verschiedene Alkalimethoxide A(i)OMe umfassen und/oder wenigstens 2 der Gemische P(i) das gleiche Alkalialkoxid A(i)OMe in verschiedenen Konzentrationen umfassen.

**Revendications**

1. Procédé intégré de préparation simultanée de n mélanges P(i) comprenant un méthoxyde de métal alcalin et du méthanol, comprenant

la fourniture de n colonnes de distillation réactive K(i) ;
la fourniture de n flux liquides aqueux H(i), un flux donné H(i) comprenant un hydroxyde de métal alcalin dissous A(i)OH, dans lequel n est un entier avec n ≥ 2 et i = 1...n ; et
la fourniture d'une colonne de rectification D ;
le procédé comprenant en outre

(a) la fourniture d'un flux G comprenant du méthanol ;
(b) la division du flux G en n flux G(i), chaque flux G(i) ayant la même composition que G ;
(c) la préparation du ou des méthoxydes de métaux alcalins comprenant l'introduction de chaque flux G(i) dans la partie inférieure d'une colonne de distillation réactive respective K(i), et l'introduction du flux liquide aqueux H(i) comprenant l'hydroxyde de métal alcalin dissous A(i)OH dans la partie supérieure de ladite colonne de distillation réactive K(i) ; et
la soumission de G(i) et H(i) dans chaque K(i) à des conditions de distillation réactive, l'obtention de n flux de tête W(i) comprenant du méthanol et de l'eau ; et l'obtention de n flux de produits de fond P(i) comprenant un méthoxyde de métal alcalin A(i)OMe et du méthanol ;
(d) l'alimentation de chaque flux W(i) dans la partie inférieure de la colonne de rectification D, l'alimentation d'un flux M comprenant du méthanol dans la colonne de rectification D ; et la soumission des n flux W(i) et M dans D à des conditions de distillation, l'obtention du flux G selon (a) comme flux de tête ;

dans lequel la colonne de rectification D est exploitée avec un rapport de reflux d'au moins 0,5:1, dans lequel la colonne de rectification D est exploitée avec une recompression de vapeurs de tête, dans lequel pour réaliser le rapport de reflux, le procédé comprend

(i) l'élimination, en plus de G, d'un flux de tête T(2) de la colonne de rectification D, le passage de ce flux T(2) à travers un condenseur V(4), l'obtention d'un flux liquide et d'un flux d'effluents gazeux T(2w) ;
(ii) l'élimination, en plus de G et T(2), d'un autre flux de tête T(1) de la colonne de rectification D, le passage de ce flux T(2) à travers un compresseur C(3), la passage du flux ainsi comprimé à travers un rebouilleur V(6), l'obtention d'un flux liquide, le rebouilleur V(6) étant un rebouilleur intermédiaire de la colonne de rectification D ;
(iii) l'alimentation des flux liquides obtenus conformément à (i) et (ii) dans la tête de la colonne de rectification D ;

ou

(i) l'élimination, en plus de G, d'un flux de tête T(2) de la colonne de rectification D, le passage dudit flux T(2) à travers un condenseur V(4), l'obtention d'un flux liquide T(2l) et d'un flux gazeux T(2g) ; le passage du flux gazeux T(2g) à travers un condenseur V(5), l'obtention d'un flux liquide T(2gl) et d'un flux d'effluents gazeux T(2w) ; et la combinaison des flux liquides T(2l) et (T2gl), l'obtention d'un flux liquide combiné T(2cl) ;
(ii) l'élimination, en plus de G et T(2), d'un autre flux de tête T(1) de la colonne de rectification D, le passage de ce flux T(2) à travers un compresseur C(3), la passage du flux ainsi comprimé à travers un rebouilleur V(6), l'obtention d'un flux liquide, le rebouilleur V(6) étant un rebouilleur intermédiaire de la colonne de rectification D ;
(iii) l'alimentation du flux liquide combiné obtenu selon (i) et du flux liquide obtenu selon (ii) dans la tête de la colonne de rectification D.

2. Procédé selon la revendication 1, dans lequel n est dans la plage de 2 à 10, de préférence dans la plage de 2 à 5, plus préférablement 2 ou 3, plus préférablement 2.

3. Procédé selon la revendication 1 ou 2, dans lequel chaque hydroxyde de métal alcalin A(i)OH est de préférence choisi dans le groupe constitué par l'hydroxyde de lithium, l'hydroxyde de sodium et l'hydroxyde de potassium, plus préférablement dans le groupe constitué par l'hydroxyde de sodium et l'hydroxyde de potassium et dans lequel un flux liquide aqueux H(i) donné comprend un hydroxyde de métal alcalin A(i)OH dissous dans l'eau, dans le méthanol, ou dans un mélange comprenant de l'eau et du méthanol, de préférence dans l'eau.

4. Procédé selon l'une quelconque des revendications 1 à 3, qui est un procédé intégré pour préparer simultanément au moins 2 mélanges P(i), de préférence 2 mélanges P(i), dans lequel le mélange P(1) comprend A(1)OMe, de préférence du méthoxyde de sodium, et du méthanol et le mélange P(2) comprend A(2)OMe, de préférence du méthoxyde de potassium, et du méthanol, le procédé comprenant

(a) la fourniture d'un flux G comprenant du méthanol ;

(b) la division du flux G en au moins deux flux G(1) et G(2), G(1) et G(2) ayant la même composition que G ;

(c.1) la préparation de A(1)OMe, de préférence de méthoxyde de sodium, comprenant

(c.1.1) l'alimentation du flux G(1) dans la partie inférieure d'une colonne de distillation réactive K(1), et l'alimentation d'un flux liquide aqueux H(1) comprenant A(1)OH dissous, de préférence de l'hydroxyde de sodium, dans la partie supérieure de la colonne de distillation réactive K(1) ;

(c.1.2) la soumission de G(1) et H(1) dans K(1) à des conditions de distillation réactive, l'obtention d'un flux de tête W(1) comprenant du méthanol et de l'eau ; et l'obtention d'un flux de produits de fond P(1) comprenant A(1)OMe, de préférence du méthoxyde de sodium, et du méthanol ;

(c.2) la préparation de A(2)OMe, de préférence du méthoxyde de potassium, comprenant

(c.2.1) l'alimentation du flux G(2) dans la partie inférieure d'une colonne de distillation réactive K(2), K(2) qui est agencée en parallèle avec K(1), et l'alimentation d'un flux liquide aqueux H(2) comprenant A(2)OH dissous, de préférence de l'hydroxyde de potassium, dans la partie supérieure de la colonne de distillation réactive K(2) ;

(c.2.2) la soumission de G(2) et H(2) dans K(2) à des conditions de distillation réactive, l'obtention d'un flux de tête W(2) comprenant du méthanol et de l'eau ; et l'obtention d'un flux de produits de fond P(2) comprenant A(2)OMe, de préférence du méthoxyde de potassium, et du méthanol ;

(d.1) l'alimentation de W(1) et W(2) dans la partie inférieure d'une colonne de rectification D et l'alimentation d'un flux M comprenant du méthanol dans la colonne de rectification D ;

(d.2) la soumission de W(1), W(2) et M dans D à des conditions de distillation, obtenir le flux G selon (a) comme flux de tête.

5. Procédé selon la revendication 4, dans lequel W(1) et W(2) sont introduits sous forme de flux gazeux dans la colonne de rectification D, de préférence à une position située entre les produits de fond et le 15$^e$ étage théorique, de préférence entre les produits de fond et le 10$^e$ étage théorique, plus préférablement entre les produits de fond et le 8$^e$ étage théorique de la colonne de rectification D.

6. Procédé selon l'une quelconque des revendications 1 à 5, dans lequel de 99 à 100 % en poids, de préférence de 99,5 à 100 % en poids, plus préférablement de 99,9 à 100 % en poids du flux M sont constitués de méthanol et éventuellement d'eau, dans lequel la quantité d'eau comprise dans le flux M est d'au plus 2 000 ppm en poids, plus préférablement d'au plus 1 500 ppm en poids, plus préférablement d'au plus 1 000 ppm en poids, dans lequel le flux M est introduit de préférence dans la partie supérieure de D, plus préférablement, au moins 4 étages théoriques à partir du sommet de D, plus préférablement entre le 4$^e$ et le 20$^e$ étage théorique à partir du sommet de D, plus préférablement entre le 6$^e$ et le 15$^e$ étage théorique à partir du sommet de D, de préférence à une température de M dans la plage allant de la température ambiante au point d'ébullition du méthanol à la pression de colonne de D, de préférence à la température ambiante.

7. Procédé selon l'une quelconque des revendications 1 à 6, dans lequel la colonne de rectification D est exploitée à un rapport de reflux dans la plage de 0,55:1 à 1,4:1, de préférence dans la plage de 0,6:1 à 1,4:1.

8. Procédé selon l'une quelconque des revendications 1 à 7, dans lequel le flux G fourni selon (a) par distillation selon (d.2) comprend du méthanol et de l'eau, dans lequel de préférence 99,95 à 100 % en poids de G sont constitués de méthanol et d'eau, et dans lequel la teneur en eau de G est d'au plus 200 ppm en poids, de préférence d'au plus 150 ppm en poids, plus préférablement d'au plus 100 ppm en poids, dans lequel plus préférablement ladite teneur en eau est dans la plage de 5 à 100 ppm en poids, plus préférablement dans la plage de 10 à 100 ppm en poids, plus préférablement dans la plage de 15 à 100 ppm en poids.

9. Procédé selon l'une quelconque des revendications 1 à 8, dans lequel selon (b), le flux G est divisé en les deux flux G(1) et G(2).

10. Procédé selon l'une quelconque des revendications 1 à 9, dans lequel, avant d'être introduit dans la colonne de distillation réactive K(1), le flux G(1) est passé à travers un compresseur C(1), réalisant ainsi une augmentation de pression de G(1) dans la plage de 0,1 à 0,8 bar(abs), de préférence dans la plage de 0,15 à 0,6 bar(abs), plus préférablement dans la plage de 0,2 à 0,4 bar(abs) ;

et/ou, de préférence et

avant d'être introduit dans la colonne de distillation réactive K(2), le flux G(2) est passé à travers un compresseur C(2), ce qui permet d'obtenir une augmentation de pression de G(2) dans la plage de 0,1 à 0,8 bar(abs), de préférence dans la plage de 0,15 à 0,6 bar(abs), plus préférablement dans la plage de 0,2 à 0,4 bar(abs) ;

**11.** Procédé selon l'une quelconque des revendications 1 à 9, dans lequel, avant d'être introduit dans la colonne de rectification D, le flux W(1) est passé à travers un compresseur C(1), réalisant ainsi une augmentation de pression de W(1) dans la plage de 0,1 à 0,8 bar(abs), de préférence dans la plage de 0,15 à 0,6 bar(abs), plus préférablement dans la plage de 0,2 à 0,4 bar(abs) ; et/ou, de préférence et

avant d'être introduit dans la colonne de rectification D, le flux W(2) passe à travers un compresseur C(2), ce qui permet d'obtenir une augmentation de pression de W(2) dans la plage de 0,1 à 0,8 bar(abs), de préférence dans la plage de 0,15 à 0,6 bar(abs), de préférence dans la plage de 0,2 à 0,4 bar(abs).

**12.** Unité de production chimique pour la mise en œuvre du procédé selon l'une quelconque des revendications 1 à 11, comprenant

- une colonne de rectification D comprenant

-- des moyens d'entrée pour alimenter le flux M dans D ;
-- dans sa partie inférieure, des moyens d'entrée pour alimenter les flux W(i) ou un ou plusieurs flux combinés de ceux-ci dans D ;
-- des moyens de sortie pour éliminer les flux T(2) et G ou un flux combiné de ceux-ci du sommet de D ;
-- au moins un condenseur, de préférence un condenseur V(4) et éventuellement un condenseur V(5) supplémentaire disposé en aval de V(4), ayant des moyens d'entrée pour recevoir le flux T(2) et des moyens de sortie pour évacuer le flux condensé T(3) et pour évacuer un flux d'effluents gazeux ;
-- des moyens d'entrée pour alimenter le flux T(3) au sommet de D ;
-- un rebouilleur de fond ;

- un dispositif de division de flux S pour diviser le flux G en n flux G(i) ;
- des moyens pour passer le flux G audit dispositif de division de flux S ;
- n colonnes de distillation réactive K(i), n $\geq$ 2 et i = 1...n ; lesdites colonnes de distillation réactive K(i) étant disposées en parallèle, chaque colonne de distillation réactive K(i) comprenant

-- dans sa partie supérieure, de préférence dans son sommet, des moyens d'entrée pour alimenter un flux H(i) dans K(i) ;
-- dans sa partie inférieure, des moyens d'entrée pour alimenter un flux G(i) dans K(i) ;
-- des moyens de sortie pour éliminer un flux W(i) du sommet de K(i) ;
-- un rebouilleur de fond ;
-- des moyens de sortie pour éliminer un flux de produits de fond de K(i) ;
-- des moyens de division de flux pour séparer un flux P(i) du flux de produits de fond éliminé de K(i) ;

- des moyens pour passer les flux G(i) aux colonnes de distillation réactive K(i) ;
- des moyens pour passer les flux W(i) à la colonne de rectification D ;
- un ou plusieurs compresseurs C(i) pour comprimer soit le flux G et/ou les flux G(i) et/ou les flux W(i) ; l'unité comprend en outre des moyens pour réaliser la recompression des vapeurs de tête obtenue de D, lesdits moyens comprenant un compresseur C(3) pour comprimer un flux T(1) prélevé du sommet de D, des moyens pour faire passer T(1) du sommet de D à C(3), un rebouilleur V(6) pour condenser ledit flux comprimé, des moyens pour passer ledit flux comprimé de (C3) à V(6), et des moyens pour alimenter le flux liquide obtenu au sommet de D, dans laquelle le rebouilleur V(6) est un rebouilleur intermédiaire de D.

**13.** Unité selon la revendication 12, comprenant n compresseurs C(i) agencés en amont de K(i) pour comprimer les flux G(i) ou comprenant n compresseurs C(i) agencés en aval de K(i) et en amont de D pour comprimer les flux W(i).

**14.** Unité selon la revendication 12 ou 13, dans laquelle les moyens d'entrée pour amener les flux W(i) ou un ou plusieurs flux combinés de ceux-ci dans D sont situés à une position entre les produits de fond et le 15$^e$ étage théorique, de préférence entre les produits de fond et le 10$^e$ étage théorique, plus préférablement entre les produits de fond et le 8$^e$ étage théorique de D ; et/ou dans laquelle les moyens d'entrée du flux M dans D sont situés dans la partie supérieure de D, de préférence à au moins 4 étages théoriques du sommet de D, de préférence entre le 4$^e$ et le 20$^e$ étage théorique du sommet de D, plus préférablement entre le 6$^e$ et le 15$^e$ étage théorique du sommet de D.

**15.** Utilisation d'une unité de production chimique selon l'une quelconque des revendications 12 à 14 ou d'un procédé selon l'une quelconque des revendications 1 à 11 pour produire simultanément n mélanges P(i) comprenant un méthoxyde de métal alcalin A(i)OMe et du méthanol, n étant un entier avec n $\geq$ 2 et i = 1...n, dans laquelle soit au moins 2 des mélanges P(i) comprennent différents méthoxydes de métaux alcalins A(i)OMe, et/ou au moins 2 des mélanges P(i) comprennent le même alcoxyde de métal alcalin A(i)OMe à différentes concentrations.

Figure 1

Figure 2

Figure 3

Figure 4

Figure 5

Figure 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20020183566 A1
- US 20080296786 A1
- WO 2013168113 A1